(19) Europäisches Patentamt
European Patent Office
Office européen des brevets

(11) Veröffentlichungsnummer: **0 207 894 B1**

# (12) EUROPÄISCHE PATENTSCHRIFT

(45) Veröffentlichungstag der Patentschrift: **21.11.91**

(21) Anmeldenummer: **86810277.3**

(22) Anmeldetag: **18.06.86**

(51) Int. Cl.5: **C07D 209/48**, C07D 317/12, C07D 405/10, C07C 249/08, C07C 251/48, A01N 37/32

Die Akte enthält technische Angaben, die nach dem Eingang der Anmeldung eingereicht wurden und die nicht in dieser Patentschrift enthalten sind.

## (54) Herbizid wirkende Derivate des N-Phenyl-3,4,5,6-tetrahydrophthalsäureimides.

(30) Priorität: **24.06.85 CH 2665/85**
**25.07.85 CH 3237/85**
**26.03.86 CH 1207/86**

(43) Veröffentlichungstag der Anmeldung:
**07.01.87 Patentblatt 87/02**

(45) Bekanntmachung des Hinweises auf die Patenterteilung:
**21.11.91 Patentblatt 91/47**

(84) Benannte Vertragsstaaten:
**BE CH DE FR GB IT LI NL**

(56) Entgegenhaltungen:
**EP-A- 0 236 916**
**DE-A- 3 109 035**
**GB-A- 2 150 929**
**US-A- 4 260 555**
**DE-A- 2 825 565**
**GB-A- 2 071 100**
**JP-A-60 152 465**

**PATENT ABSTRACTS OF JAPAN, unexamined applications, C Field, Band 8, Nr. 251, 16. November 1984 THE PATENT OFFICE JAPANESE GOVERNMENT Seite 21 C 252**

**CHEMICAL ABSTRACTS, Band 87, Nr. 1, 4. Juli 1977, Columbus, Ohio, USA; WAGNER G. et al.: "Synthesis of N-(amidinobenzyl)- and N-(amidinophenyl)phthalimides and -1-oxoindolines" Seite 464, Spalte 1, Zusammenfassung-Nr. 5 753d**

**CHEMICAL ABSTRACTS, Band 90, 12. Februar - 25. Februar 1979, Columbus, Ohio, USA; BOLLINGER, FREDERIC G. et al.: "Substituted phthalimides for regulating the growth of plants" Seite 139, Spalte 1, Zusammenfassung-Nr. 67 726t**

(73) Patentinhaber: **CIBA-GEIGY AG**
**Klybeckstrasse 141**
**CH-4002 Basel(CH)**

(72) Erfinder: **Pissiotas, Georg, Dr.**
**Am Sonnenrain 71**
**W-7850 Lörrach(DE)**
Erfinder: **Böhner, Beat, Dr.**
**Hügelweg 3**
**CH-4102 Binningen(CH)**

EP 0 207 894 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Derivate den N-Phenyl-3,4,5,6-tetrahydrophthalsäureimides der Formel I mit herbizider und den Pflanzenwuchs regulierender Wirkung, sowie die Herstellung dieser neuen Verbindungen. Ferner betrifft sie Mittel, welche die neuen Verbindungen enthalten sowie deren Verwendung zur selektiven Bekämpfung von Unkräutern oder zur Regulierung des Pflanzenwuchses.

Die neuen Derivate des N-Phenyl-3,4,5,6-tetrahydrophthalsäureimides entsprechen der Formel I

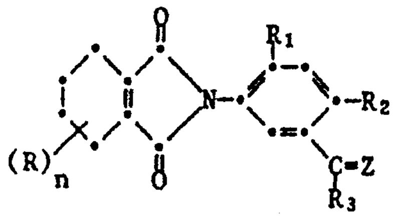

(I),

worin

R $C_1$-$C_3$-Alkyl,

n Null, eins oder zwei,

$R_1$ Fluor

$R_2$ Wasserstoff oder Halogen,

Z Sauerstoff oder einen Oximrest =N-O-Q, der Rest

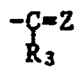

kann auch einen Ketalrest

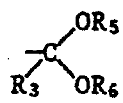

darstellen,

$R_3$ falls Z einen Oximrest darstellt, Methyl oder Cyan,

$R_3$ wenn Z Sauerstoff oder einen Ketalrest darstellt, Cyan, $C_1$-$C_4$-Alkyl, $C_1$-$C_4$-Halogenalkyl oder einen Rest -$CH_2YR_4$

Y Sauerstoff oder Schwefel,

$R_4$ $C_1$-$C_4$-Alkyl, $C_3$-$C_4$-Alkenyl, $C_3$-$C_4$-Alkinyl, $C_3$-$C_7$-Cycloalkyl, Benzyl oder Phenyl, wobei der Phenylring unsubstituiert oder durch $C_1$-$C_4$-Alkyl, $C_1$-$C_4$-Alkoxy, $C_1$-$C_4$-Halogenalkyl, $C_1$-$C_4$-Halogenalkoxy, Nitro oder Cyan substituiert sein kann,

$R_5$ und $R_6$ unabhängig voneinander je $C_1$-$C_4$-Alkyl, $C_1$-$C_4$-Halogenalkyl, oder $C_2$-$C_8$-Alkoxyalkyl

$R_5$ und $R_6$ zusammen eine Aethylenbrücke, eine Propylenbrücke oder einen über benachbarte Kohlenstoffatome gebundenen Cyclohexylrest bedeuten, wobei die Propylenbrücke und der Cyclohexlyrest bis zu dreimal durch $C_1$-$C_4$-Alkylreste substituiert sein können, während die Aethylenbrücke ein- oder zweimal durch $C_1$-$C_4$-Alkyl, $C_1$-$C_4$-Halogenalkyl, unsubstituiertes oder durch Halogen oder $C_1$-$C_4$-Alkyl substituiertes Phenyl oder Benzyl einen Rest -$CH_2YH$ oder -$CH_2YR_4$ substituiert sein kann,

und

Q Wasserstoff

einen $C_1$-$C_{10}$-Alkylrest, der durch Sauerstoff, Schwefel -CO-, -CONH- oder -NH- unterbrochen oder der durch Halogen oder Cyan substituiert sein kann,

einen $C_3$-$C_8$-Alkenyl- oder Halogenalkenylrest,

einen $C_3$-$C_8$-Alkinylrest,

einen gegebenenfalls durch Halogen substituierten $C_3$-$C_7$- Cycloalkylrest,

einen Phenyl-$C_1$-$C_4$-Alkylrest dessen Phenylkern durch Halogen, $C_1$-$C_4$-Alkyl, $C_1$-$C_4$-Alkoxy, $C_1$-$C_4$-Halogenalkyl, $C_1$-$C_4$-Halogenalkoxy, Nitro oder cyan substituiert sein kann,

einen $C_1$-$C_4$-Alkoxycarbonylrest,
einen $C_1$-$C_4$-Alkylthiocarbonylrest,
einen Benzoyl- Furoyl- oder Thienoylrest, der im Ring unsubstituiert oder durch Halogen. $C_1$-$C_4$-Alkyl, $C_1$-$C_4$-Alkoxy, $C_1$-$C_4$-Halogenalkoxy, Nitro oder Cyan substituiert ist,
einen $C_1$-$C_4$-Alkylsulfonylrest, einen Sulfonamido- oder Carbamoyl-rest, der unsubstituiert oder durch $C_1$-$C_4$-Alkylrest(e) substituiert ist,
ein Metallion oder ein quaternisiertes Ammoniumion,
einen über COO- resp. CSO-gebundenen Phenyl- oder Benzylrest bedeutet.

Die Erfindung betrifft auch die Stereoisomeren sowie die optisch aktiven Enantiomere welche diese Verbindungen aufweisen können.

In der Formel I ist unter Halogen, Fluor, Chlor, Brom oder Iod zu verstehen, vor allem Fluor, Chlor oder Brom.

Der Ausdruck Alkyl allein oder als Teil eines Substituenten umfasst verzweigte und geradkettige Reste. Beispiele sind Methyl, Aethyl, n-Propyl, Isopropyl, n-Butyl, sec. Butyl, tert.Butyl, Isobutyl sowie die höheren Homologen, Pentyl, Hexyl, Heptyl, Octyl etc. samt ihren Isomeren. Sinngemäss enthalten Alkanoyle und Cyanalkyle ein zusätzliches Kohlenstoffatom.

Die Alkenyl- und Alkinylreste können ebenfalls verzweigt oder geradkettig sein und umfassen beispielsweise die Gruppen Allyl, Methallyl, Butenyl, Propanyl oder Butinyl, Propinyl.

Bevorzugte Carbonsäureester und -thioester sind $C_1$-$C_4$-Alkyl, $C_3$-$C_4$-Alkenyl- oder $C_3$-$C_4$-Alkynylester.

Carbonsäureamide und Sulfonsäureamide bedeuten neben $-CONH_2$ bzw. $-SO_2-NH_2$ auch monoalkylsubstituierte oder symmetrisch oder unsymmetrisch dialkylsubstituierte Amide, wobei die Alkylgruppen $C_1$-$C_4$-Kohlenstoffe enthalten.

Die Definition von Q umfasst 5- gliedrige heterocyclische Carboxylverbindungen mit einem Ring-Heteroatom aus der Reihe O oder S. Beispiele sind die Radikale der Furancarbonsäure und Thiophencarbonsäure.

Cycloalkylgruppen sind Cyclopropyl, Cyclobutyl, Cyclopentyl, Cyclohexyl und Cycloheptyl. Cycloaliphatische Reste entsprechen diesen Ringsystemen, können daneben aber noch, je nach Möglichkeit, eine oder mehrere Doppelbindungen enthalten.

Als Metallionen sind Kationen der I. bis IV. Gruppen des Periodensystems sowie Schwermetallsalze zu verstehen. Beispiele sind Na, K, Ca, Mg, Al, Zn, Cu, Fe, Mn, Co, Ni.

Quaternäre Ammoniumionen enthalten als gleiche oder verschiedene Substituenten Wasserstoff, $C_1$-$C_{12}$-Alkyl, Niederhydroxyalkyl, Benzyl, Amino, Di-$C_1$-$C_4$-alkylamino oder formen aus zwei Valenzen und dem N-Atom einen 5- oder 6-gliedrigen heterocyclischen Ring mit gegebenenfalls einem weiteren Heteroatom N, O oder S.

Die neuen Derivate des N-Phenyl-3,4,5,6-tetrahydrophthalsäureimides der Formel I zeichnen sich durch eine herbizide Wirkung aus und können zur Bekämpfung von Unkräutern eingesetzt werden. Je nach der Dosierung wirken sie als totale oder selektive Herbizide, und können zur Kontrolle von Unkräuter in Kulturen von Nutzpflanzen verwendet werden.

Diese Verbindungen besitzen ebenfalls eine den Pflanzenwuchs regulierende Wirkung und können zur Hemmung des Pflanzenwuchses verwendet werden.

Als besonders aktiv haben sich die Keto-Verbindungen erwiesen, welche der Formel Ia entsprechen

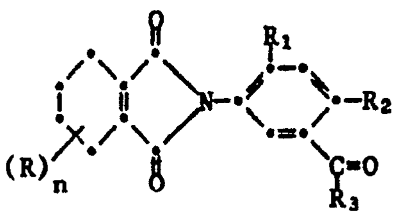

(Ia),

worin
$R_3$ $C_1$-$C_4$-Alkyl oder einen Rest-$CH_2YR_4$ bedeutet während n, R, $R_1$, $R_2$ und $R_4$ die unter der Formel I gegebene Bedeutung haben, insbesondere
2-Chlor-4-fluor-5-(N-3,4,5,6-tetrahydrophthalsäureimido)-acetophenon,
4-Fluor-5-(N-3,4,5,6-tetrahydrophthalsäureimido)-acetophenon,
2-Brom-4-fluor-5-(N-3,4,5,6-tetrahydrophthalsäureimido)-acetophenon.

Ebenfalls sehr aktiv sind die Ketal-Verbindungen der Formel Ib

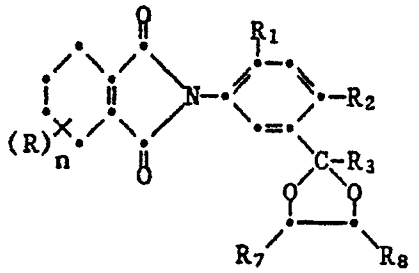

(Ib),

worin $R_3$, $C_1$-$C_4$ Alkyl und $R_7$ und $R_8$ unabhängig voneinander je Wasserstoff oder $C_1$-$C_4$-Alkyl bedeuten, während n, R, $R_1$ und $R_2$ die unter der Formel I gegebene Bedeutung haben, insbesondere
N-[2-Fluor-4-chlor-5-(2-methyl-1,3-dioxolan-2-yl)-phenyl]-3,4,5,6-tetrahydro-phthalsäureimid,
N-[2-Fluor-4-chlor-5-(2,4-dimethyl-1,3-dioxolan-2-yl)-phenyl]-3,4,5,6-tetrahydro-phthalsäureimid,
N-[2-Fluor-4-chlor-5-(4-äthyl-2-methyl-1,3-dioxan-2-yl)-phenyl]-3,4,5,6-tetrahydro-phthalsäureimid,
N-[2-Fluor-5-(2-methyl-1,3-dioxolan-2-yl)-phenyl]-3,4,5,6-tetrahydro-phthalsäureimid,
N-[2,4-Difluor-5-(2,4-dimethyl-1,3-dioxolan-2-yl-)-phenyl]-3,4,5,6-tetrahydro-phthalsäureimid,
N-[2,4-Difluor-5-(2,4,5-trimethyl-1,3-dioxolan-2-yl)-phenyl]-3,4,5,6-tetrahydro-phthalsäureimid,
N-[4-Fluor-5-(4-äthyl-2-methyl-1,3-dioxolan-2-yl)-phenyl]-3,4,5,6-tetrahydro-phthalsäureimid und
N-[2-Fluor-4-chlor-5-(2,4,5-trimethyl-1,3-dioxolan-2-yl)-phenyl]-3,4,5,6-tetrahydro-phthalsäureimid.

Weiterhin sehr gute Wirkung zeigen die Oxim-Verbindungen der Formel (Ic)

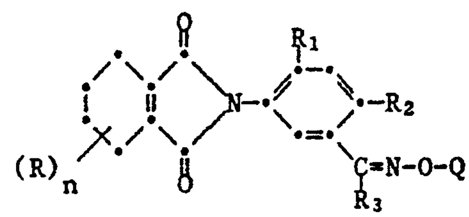

(Ic)

worin
n $R_1$ und R die unter der Formel I gegebene Bedeutung haben, $R_3$ Methyl oder Cyan bedeutet und Q Wasserstoff einen $C_1$-$C_{10}$-Alkylrest, der durch Sauerstoff, Schwefel oder NH- unterbrochen, oder der durch Halogen oder Cyan substituiert sein kann; einen $C_3$-$C_8$-Alkenyl oder Halogenalkenylrest; einen $C_3$-$C_8$-Alkinylrest; einen Phenyl-$C_1$-$C_4$-alkylrest, dessen Phenylkern durch Halogen, $C_1$-$C_4$-Alkyl, $C_1$-$C_4$-Alkoxy, $C_1$-$C_4$-Halogenalkyl, $C_1$-$C_4$-Halogenalkoxy, Nitro oder Cyan substituiert sein kann; einen $C_1$-$C_4$-Alkylcarbonylrest; einen $C_1$-$C_4$-Alkylthiocarbonylrest; einen $C_1$-$C_4$-Alkylsulfonylrest oder einen $C_1$-$C_4$-Alkylcarbamoylrest bedeuten, insbesondere
2-Chlor-4-fluor-5-(N-3,4,5,6-tetrahydrophthalsäureimido)-acetophenonoxim,
4-Fluor-5-(N-3,4,5,6-tetrahydrophtalsäureamido)-acetophenonoxim,
2-Chlor-4-fluor-5-(N-3,4,5,6-tetrahydrophthalsäureimido)-acetophenonoxim-(methoxy-carbonyl-äth-1-yl)-äther,
4-Fluor-5-(N-3,4,5,6-tetrahydrophthalsäureimido)-acetophenonoxim-(methoxy-carbonyl-äth-1-yl)-äther,
2-Chlor-4-fluor-5-(N-3,4,5,6-tetrahydrophthalsäureimido)-acetophenonoxim-methyl-äther,
2-Chlor-4-fluor-5-(N-3,4,5,6-tetrahydrophthalsäureimido)-acetophenonoxim-äthyl-äther,
2-Chlor-4-fluor-5-(N-3,4,5,6-tetrahydrophthalsäureimido)-acetophenonoxim-n-butyl-äther,
2-Chlor-4-fluor-5-(N-3,4,5,6-tetrahydrophthalsäureimido)-acetophenonoxim-t-butyl-äther,
2-Chlor-4-fluor-5-(N-3,4,5,6-tetrahydrophthalsäureimido)-acetophenonoxim-benzyl-äther,
2-Chlor-4-fluor-5-(N-3,4,5,6-tetrahydrophthalsäureimido)-acetophenonoxim-(2-chlor-benzyl)-äther,
2,4-Difluor-5-(N-3,4,5,6-tetrahydrophthalsäureimido)-acetophenonoxim-(2-chlorbenzyl)-äther.

Schliesslich haben auch diejenigen Oximverbindungen der Formel Ic eine gute Wirkung, in denen n Null, $R_3$ Cyan und Q Wasserstoff oder $C_1$-$C_{10}$-Alkyl unsubstituiert oder durch Halogen substituiert; $C_3$-$C_8$-Alkenyl oder Halogenalkenyl, Benzoyl oder Halogenbenzoyl bedeuten, während $R_1$ und $R_2$ die unter der Formel 1 gegebene Bedeutung haben, besonders
Methoximino-[2-chlor-4-fluor-5-(N-3,4,5,6-tetrahydrophthalsäureimido)]-benzoyl-cyanide,
Aethoximino-[2-chlor-4-fluor-5-(N-3,4,5,6-tetrahydrophthalsäureimido)]-benzoyl-cyanide,
Isopropoximino-[2-chlor-4-fluor-5-(N-3,4,5,6-tetrahydrophthalsäureimido)]-benzoyl-cyanide,
sec.-Butoximino-[2-chlor-4-fluor-5-(N-3,4,5,6-tetrahydrophthalsäureimido)]-benzoyl-cyanide und

Methoxycarbonyl-äth-1-oximino-2-chlor-4-fluor-5-(N-3,4,5,6-tetrahydrophthalsäureimido)-benzoyl-cyanide.

Die erfindungsgemässen Derivate des N-Phenyl-3,4,5,6-tetrahydrophthalsäureimides sind neu und zeichnen sich durch gute Herbizidwirkung aus. Sie eignen sich als Selektivherbizide in Kulturen von mono- und dikotylen Nutzpflanzen.

In der GB-A 2 071 100 sind 5-(N-tetrahydrophthalimido)-benzoesäureester mit herbizider und selektiv-herbizider Wirkung beschrieben. Aus der GB-A 2 150 929 sind einige N-Phenyl-tetrahydrophthalimido-ketone und -oximäther mit herbizider Wirkung bekannt, die sich von den erfindungsgemässen Verbindungen durch die Substitution am Phenylring unterscheiden. In der JP-A 60-152 465 sowie in der nicht vorpublizierten EP-A 236 916 sind ferner N-Phenyl-tetrahydrophthalimido-aldoximäther mit herbizider und selektiv-herbizider Wirkung beschrieben.

Die Herstellung der erfindungsgemässen N-Phenyl-3,4,5,6-tetrahydrophthalsäureimide der Formel I erfolgt nach an sich bekannten Verfahren.

Das erfindungsgemässe Verfahren zur Herstellung der Derivate des N-Phenyl-3,4,5,6-Tetrahydrophthalsäureimides der Formel I besteht darin, dass man Anhydrid einer 3,4,5,6-Tetrahydrophthalsäure der Formel II

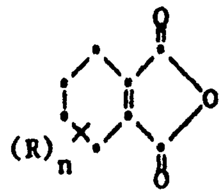

(II),

worin n und R die unter der Formel I gegebene Bedeutung haben, in einem inerten organischen Lösungs- oder Verdünnungsmittel, gegebenenfalls in Gegenwart einer geringen Menge eines wasserentziehenden Mittels, mit der äquimolaren Menge eines 3-Aminophenons oder Oxims der Formel V

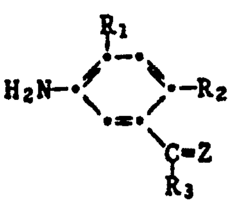

(V),

worin $R_1$, $R_2$, $R_3$ und Z die unter der Formel I gegebene Bedeutung hat, umsetzt.

Die neuen Keto- und Ketal-Derivate des N-Phenyl-3,4,5,6-Tetrahydrophthalsäureimides der Formel Ia werden erfindungsgemäss hergestellt, indem man das Anhydrid einer 3,4,5,6-Tetrahydrophthalsäure der Formel II

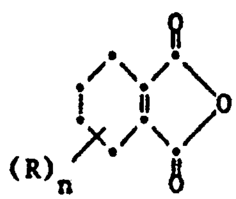

(II)

worin R und n die unter der Formel I gegebene Bedeutung haben, in einem inerten organischen Lösungs- oder Verdünnungsmittel, gegebenenfalls in Gegenwart einer geringen Menge eines wasserentziehenden Mittels, mit der äquimolaren Menge eines 3-Aminophenones der Formel III

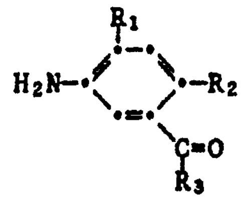

(III)

worin $R_1$, $R_2$ und $R_3$ die unter der Formel I gegebene Bedeutung haben, umsetzt und falls Z eine von Sauerstoff verschiedene Bedeutung hat, dieses Produkt in einem inerten Lösungsmittel in Gegenwart einer Säure mit einem Alkohol oder einem Diol der Formeln IV resp. IVa

$HO\text{-}R_5$ (IV) resp. $HO\text{-}R_5\text{-}R_6\text{-}OH$ (IVa)

worin $R_5$ und $R_6$ die unter der Formel I gegebene Bedeutung haben, umsetzt.

Als Lösungs- oder Verdünnungsmittel eignen sich für diese Umsetzung höhersiedende Kohlenwasserstoffe, niedere Alkansäuren sowie deren Ester, höhersiedende Ketone und Aether. Als Beispiele seien Toluol, Xylol, Essigsäure, Essigsäureäthylester, Isopropyläther, Tetrahydrofuran, Methyläthylketon, sowie Dimethylformamid erwähnt.

Die Umsetzung findet bei einer Temperatur, die zwischen der Raumtemperatur und dem Siedepunkt des Reaktionsgemisches liegt, statt.

Zur Beschleunigung der Reaktion kann eine geringe Menge eines wasserentziehenden resp. wasserabsorbierenden Mittels zugegeben werden, wie beispielsweise Schwefelsäure oder einer organischen Sulfonsäure, eines Salzes, wie Natriumacetat eines Anhydrides, wie Phosporpentoxid.

Das 3-Aminophenon der Formel III kann z.B. entsprechend der folgenden Synthese hergestellt werden:

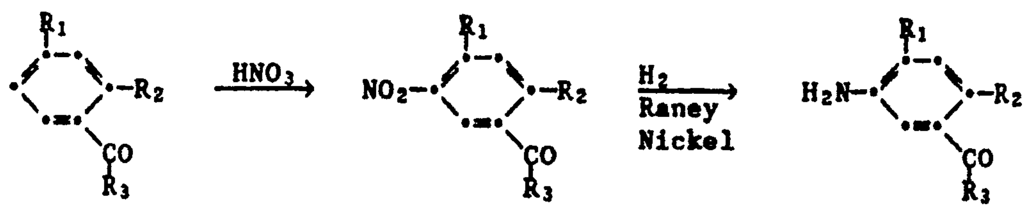


Ein im Phenylkern entsprechend substituiertes Phenon wird mit einem Salpetersäuregemisch nitriert und das entstandene 3-Nitrophenon anschliessend mit Wasserstoff z.B. in Gegenwart von Raney Nickel zum 3-Amino-phenon reduziert. Das bin wird anschliessend mit einem 3,4,5,6-Tetrahydrophthalsäureanhydrid kondensiert, entsprechend dem erfindungsgemässen Verfahren.

5-(N-3,4,5,6-Tetraphthalsäureimido)-phenonderivate der Formel I, in denen $R_3$ einen Rest $-CH_2\text{-}YR_4$ und Z zusammen mit dem Kohlenstoffatom einen Rest

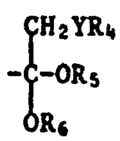

bedeutet, wobei n, R, $R_1$, $R_2$, $R_4$, $R_5$, $R_6$ und Y die unter Formel I gegebene Bedeutung haben, können beispielsweise durch die im folgenden Schema wiedergegebenen Reaktionen hergestellt werden.

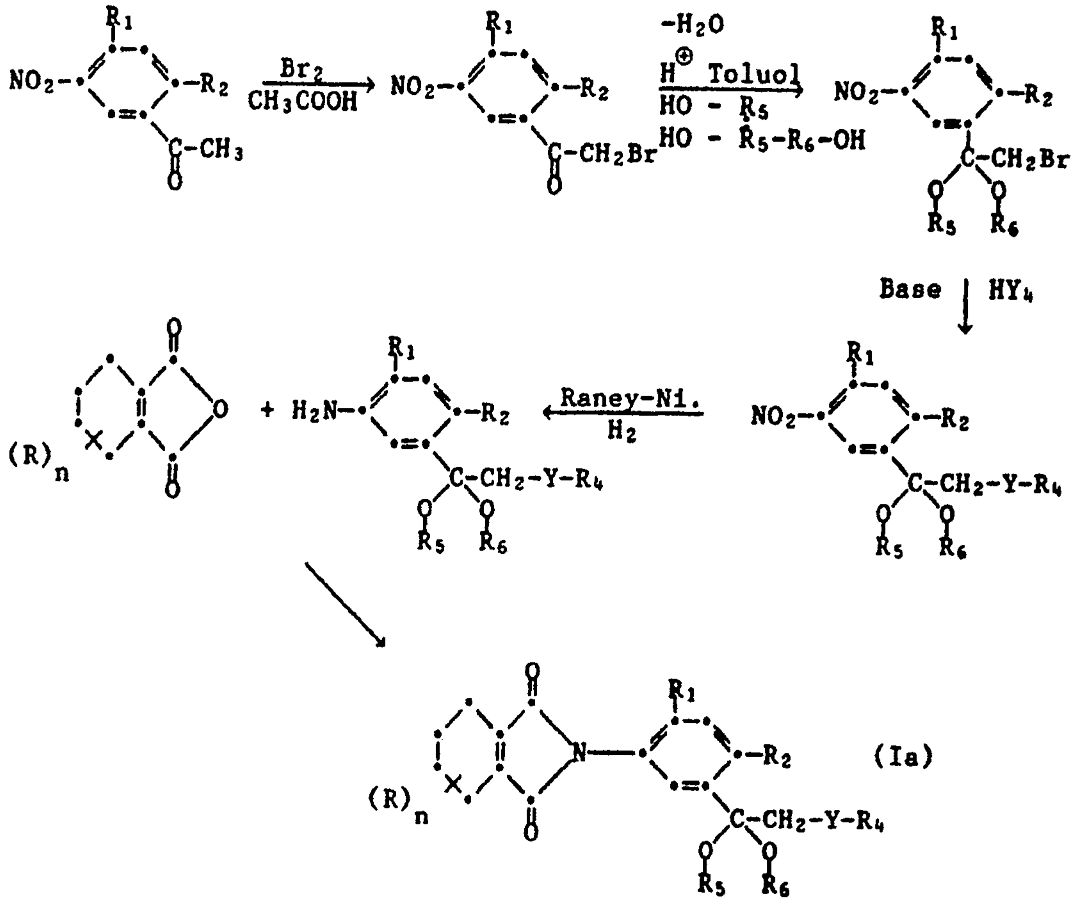


(Ia)

Ein durch $R_1$ und $R_2$ substituiertes 3-Nitro-acetophenon wird mit Brom in Essigsäure zum α-Brom-3-nitroacetophenon bromiert, welches anschliessend in einem inerten Lösungsmittel in Gegenwart einer Säure mit Alkohol oder einem Diol der Formel IV zua α-Brom-3-nitro-acetophenon-ketal umsgesetzt. Dieses Ketal wird anschliessend mit einem Reduktionsmittel, wie z.B. Wasserstoff in Gegenwart von Raney-Nickel oder Zinn und verdünnter Salzsäure oder Lithiumaluminiumhydrid zum 3-Aminoacetophenonketal reduziert, welches anschliessend erfindungsgemäss mit dem Anhydrid einer 3,4,5,6-Tetrahydrophthalsäure der Formel II umgesetzt wird.

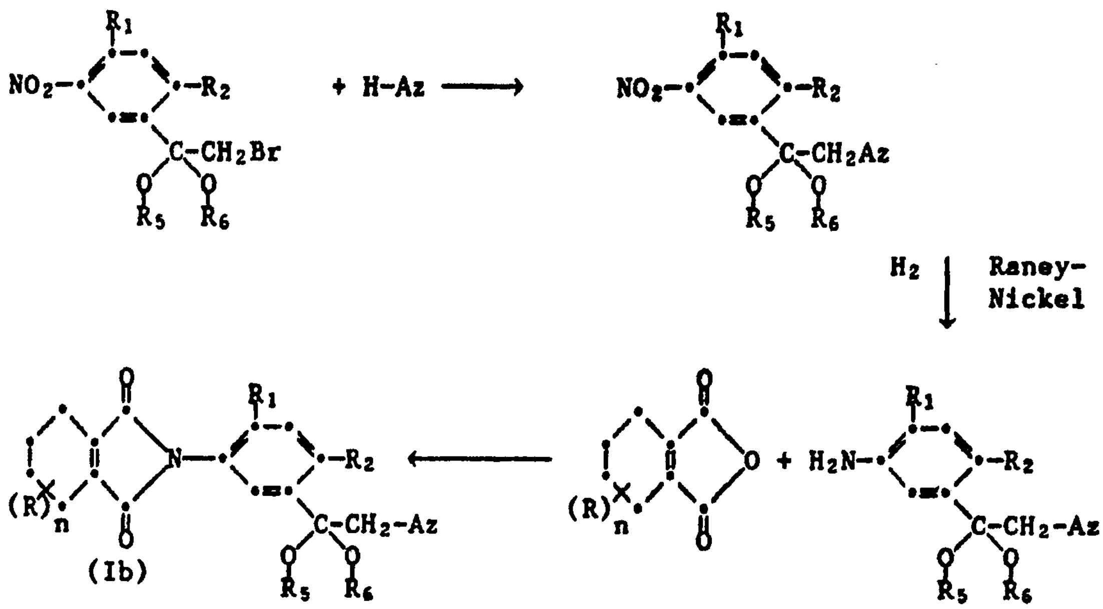


(Ib)

In diesen Formeln bedeutet Az den Imidazol-1-yl, 1,2,4-Triazol-1-yl oder 1,3,4-Triazol-1-yl-rest, n, R, $R_1$, $R_2$, $R_5$ und $R_6$ haben die unter der Formel I gegebene Bedeutung.

Ein durch $R_1$, $R_2$, $R_5$ und $R_6$ entsprechend substituiertes α-Brom-3-nitro-acetophenon-ketal wird mit Imidazol, 1,2,4- oder 1,3,4-Triazol zum α-Azolyl-3-nitro-acetophenon-ketal kondensiert. Dieses Produkt wird dann reduziert z.B. mit Wasserstoff und Raney-Nickel oder Zinn und verdünnter Salzsäure oder Lithiumaluminium oder Borhydrid, worauf das entsprechende α-Azolyl-3-amino-acetophenon-ketal entsteht, welches in erfindungsgemässer Weise mit dem Anhydrid einer 3,4,5,6-Tetrahydro-phthalsäure der Formel II umgesetzt wird.

Die neuen Oximderivate des N-Phenyl-3,4,5,6-tetrahydrophthalsäureimides der Formel Ic werden erfindungsgemäss hergestellt, indem man ein Salz der Formel VII

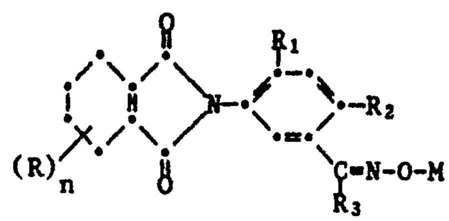

(VII),

in welcher M ein Alkalimetall- oder Erdalkalimetallkation darstellt und n, R, $R_1$, $R_2$ und $R_3$ die oben angegebene Bedeutung haben, mit einem reaktionsfähigen Ester der Formel VIII umsetzt

$$Y - Q \quad (VIII)$$

worin Q die unter Formel I gegebene Bedeutung hat und Y einen organischen oder anorganischen Säurerest darstellt.

Als Salze eines Oxims der Formel VII sind insbesondere die Natrium- und Kaliumsalze geeignet. Die Umsetzung des Oxims der Formel VII mit dem reaktionsfähigen Ester der Formel VIII wird vorteilhaft in einem inerten organischen Lösungsmittel durchgeführt. Besonders geeignet sind polare Lösungsmittel, wie Acetonitril, Dimethylformamid, Aceton, Dimethylacetamid, Methylpyrrolidon und Dimethylsulfoxid. Die Reaktanten werden in der Regel in äquimolarer Menge eingesetzt. Es kann jedoch auch zum vollständigen Ablauf der Reaktion der eine oder der andere Reaktionspartner im Ueberschuss eingesetzt werden. Als reaktionsfähige Säurereste sind die Halogenide aber auch Sulfonsäurereste, z.B. von Methyl-, Aethyl-, Phenyl- oder Toluolsulfonsäure besonders geeignet. Die Umsetzung wird vorteilhafterweise bei eine Temperatur von 60-90°C durchgeführt. Wenn ein anderes Lösungsmittel, z.B. Toluol ode Chlorbenzol verwendet wird, so geschieht die Umsetzung bei höherer Temperatur und mit einer längeren Reaktionsdauer.

Gemäss der US Patentschrift Nr. 4 260 555 wird der Rest Q mit einem Sulfonsäurerest verestert und der so erhaltene Ester der Formel IX

$$Z - SO_3 - Q \quad (IX)$$

worin Z einen niederen Alkylrest oder einen durch Niederalkyl oder Halogen substituierten Phenylrest bedeutet mit dem Oxim der Formel VII oder einem Salz davon umsetzt.

Die Oxime der Formel Ic können in bekannter Weise durch Umsetzung der entsprechenden Ketone mit Hydroxylamin hergestellt werden. Die hierfür benötigten Ketone können ihrerseits z.B. durch die in den folgenden Schema dargestellten Reaktionsfolgen erhalten werden:

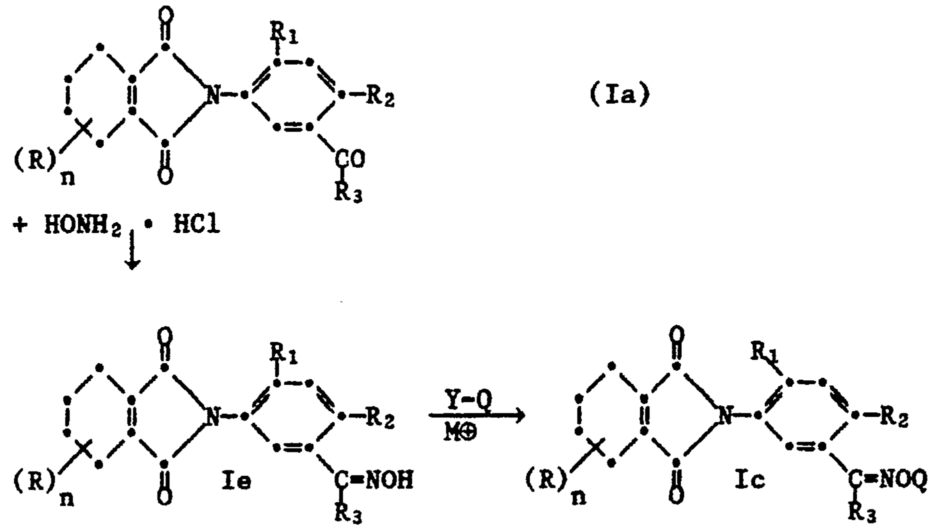


Ein im Phenylkern entsprechend substituiertes 3-(N-3,4,5,6-Tetrahydrophthalsäureimido)-phenon wird mit Hydroxylamin-hydrochlorid behandelt, wobei ein Oximderivat des N-Phenyl-3,4,5,6-tetrahydrophthalsäureimides entsprechend der Formel Ie entsteht, in welchem Q Wasserstoff bedeutet. Will man andere Derivate dieser Verbindungen herstellen, in denen Q eine von Wasserstoff verschiedene Bedeutung hat, so wird das obige Oxim zuerst mit einer Alkalimetall- oder Erdalkalimetallbase behandelt und dann mit einem reaktionsfähigen Ester A-Q umgesetzt, worin A z.B. ein Halogenid oder einen Alkyl- oder Tolylsulfonylsäurerest bedeutet.

Schliesslich Kann man die neuen Oximderivate der Formel I auch durch weitere, durch das untenstehende Schema veranschaulichte Reaktionsfolge herstellen.

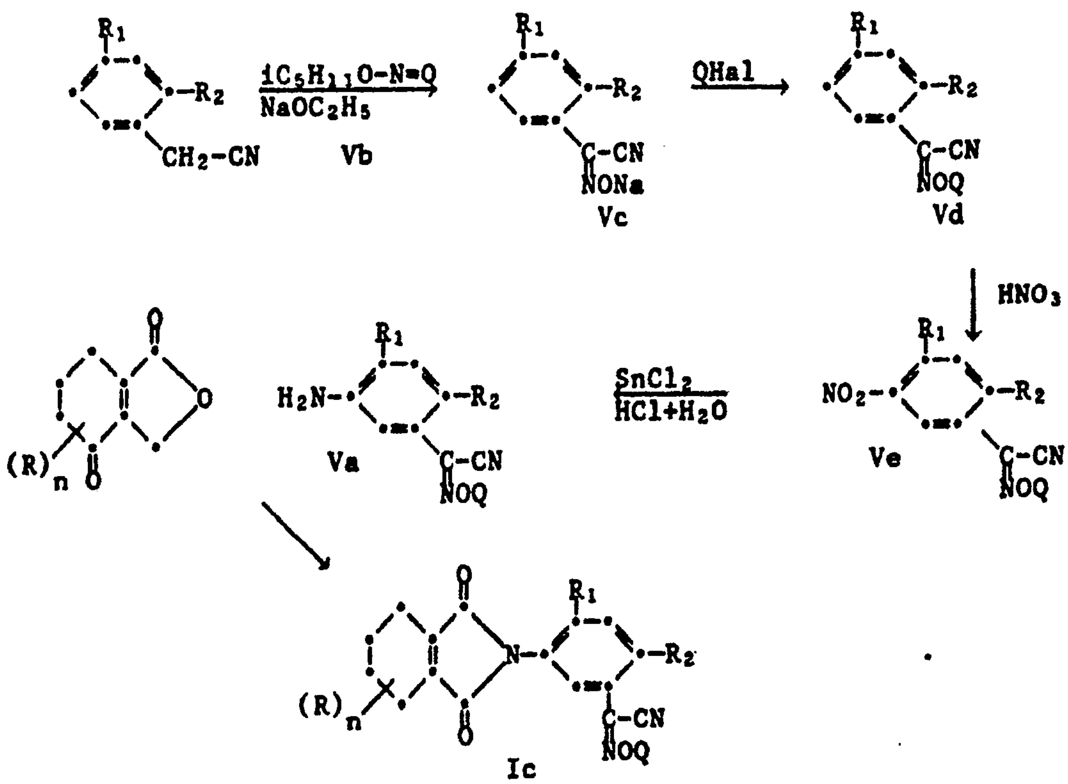


Ein in 2- und 4-Stellung durch $R_1$ und $R_2$-substituiertes Cyanobenzyl der Formel Vb wird in Gegenwart von Natriumäthylat mit Isopentylnitrit behandelt worauf sich das Cyanoacetoxim-Natrium-Salz der Formel Vc bildet, welches mit einem Halogenid der Formel QXHal veräthert wird. Der erhaltene Cyanoacetoxim-äther der Formel Vd wird anschliessend mit Salpetersäure nitriert und die Nitrogruppe mit Zinnchlorid in

wässriger Salzsäure reduziert. Man erhält so einen 3-Amino-Phenylcyanoacetoximäther welcher mit 3,4,5,6-Tetrahydrophthalsäureanhydrid zur Vebindung der Formel I kondensiert wird.

In den obigen Formeln haben n, R, $R_1$, $R_2$ und Q die unter der Formel I gegebene Bedeutung, X ist ein Halogenatom.

Ein weiteres Verfahren zur Herstellung der Oximderivate der N-Phenyl-3,4,5,6-tetrahydrophthalsäureimides der Formel Ic ist dadurch gekennzeichnet, dass man ein Anhydrid einer 3,4,5,6-Tetrahydrophthalsäure der Formel II in einem inerten organischen Lösungs- oder Verdünnungsmittels, gegebenenfalls in Gegenwart einer Base mit einem 3-Aminophenoxim-äther der obigen Formel Va umsetzt.

Die Herstellung der 3-Amino-phenoximäther der Formel Va gelingt auch ausgehend von Benzylcyaniden entsprechend dem folgenden Schema:

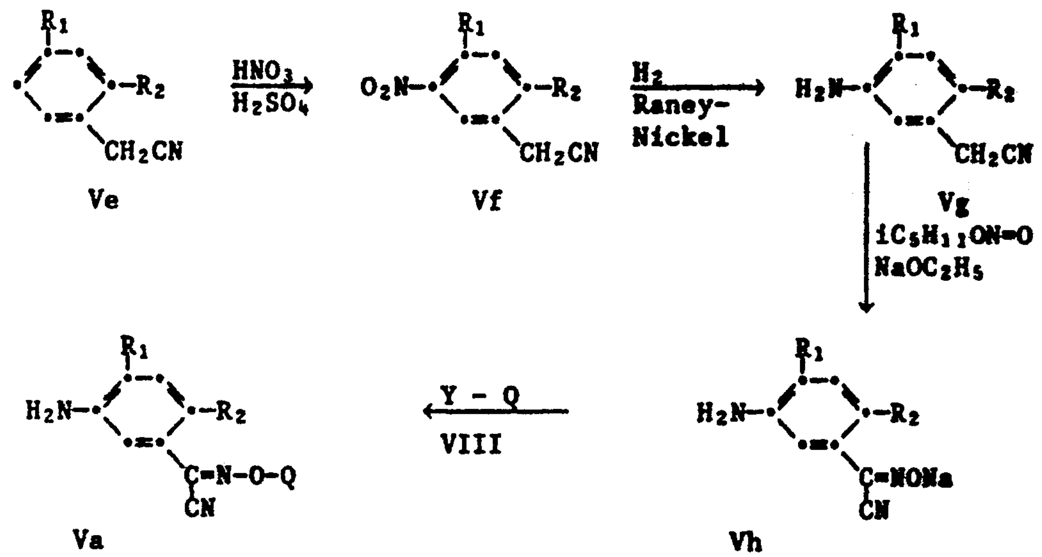

Ve Vf Vg Vh Va

Ein entsprechend substituiertes Benzylcyanid der Formel Ve wird in konz. Schwefelsäure nitriert. Das entstandene 3-Nitrobenzylcyanid der Formel Vf wird anschliessend in Gegenwart eines Katalysators mit Wasserstoff hydriert zum 3-Aminobenzylcyanid der Formel Vg. Durch Behandeln mit Isopentylnitrit in Gegenwart von Natrium und Aethanol entsteht das Na-Salz des 3-Amino-benzoylcyanid-oxims der Formel Vh, welches mit einem Ester der Formel VIII zum 3-Aminophenoximäther der Formel Va umgesetzt wird. Dieses kann wie oben beschrieben mit dem 3,4,5,6-Tetrahydrophthalsäureanhydrid der Formel II kondensiert werden.

Die 3-Aminophenoxim-äther der Formel Va sind neue Produkte. Sie und ihre Herstellung bilden ebenfalls einen Gegenstand dieser Erfindung.

Die Wirkstoffe der Formel I werden in der Regel mit Aufwandmengen von 0,005 bis 4 Kg/ha insbesondere 0,001 bis 1 kg/ha erfolgreich eingesetzt.

Bei geringeren Aufwandmengen zeichnen sich die Verbindungen der Formel I durch gute selektiv-wuchshemmende und selektiv-herbizide Eigenschaften aus, die sie ausgezeichnet zum Einsatz in Kulturen von Nutzpflanzen, insbesondere in Getreide, Baumwolle, Soja, Mais und Reis, befähigen. Es werden dabei teilweise auch Unkräuter geschädigt, welchen bisher nur mit Totalherbiziden beizukommen war.

Die Wirkungsart dieser Wirkstoffe ist unüblich. Viele sind translozierbar, d.h. sie werden von der Pflanze aufgenommen und an andere Stellen transportiert, wo sie dann zur Wirkung kommen. So gelingt es beispielsweise, durch Oberflächenbehandlung perennierende Unkräuter bis in die Wurzeln zu schädigen. Die neuen Verbindungen der Formel I wirken bereits bei - im Vergleich zu anderen Herbiziden und Wuchsregulatoren - sehr geringen Aufwandmengen.

Die Verbindungen der Formel I haben ausserdem starke pflanzenwuchshemmende, Eigenschaften. Es werden sowohl Monokotyledonen als auch Dikotyledonen in ihrem Wachstum beeinträchtigt.

So können z.B. die in der Landwirtschaft in tropischen Gegenden häufig als "cover crops" (Bodenbedecker) angepflanzten Leguminosen durch die Verbindungen der Formel I in ihrem Wachstum selektiv gehemmt werden, so dass zwar die Bodenerosion zwischen den Kulturpflanzen verhindert wird, die "cover crops" jedoch nicht zur Konkurrenz für die Kultur werden können.

Eine Hemmung des vegetativen Wachstums erlaubt bei vielen Kulturpflanzen eine dichtere Anpflanzung der Kultur, so dass ein Mehrertrag, bezogen auf die Bodenfläche, erzielt werden kann.

Ein weiterer Mechanismus der Ertragssteigerung mit Wachstumshemmern beruht darauf, dass die Nährstoffe in stärkerem Masse der Blüten- und Fruchtbildung zugute kommen, während das vegetative Wachstum eingeschränkt wird.

Bei grösseren Aufwandmengen werden alle getesteten Pflanzen in ihrer Entwicklung so geschädigt, dass sie absterben.

Die Erfindung betrifft auch herbizide und pflanzenwachstumsregulierende Mittel, welche einen neuen Wirkstoff der Formel I enthalten, sowie Verfahren zur pre- und post-emergenten Unkrautbekämpfung und zur Hemmung des Pflanzenwuchses von monokotylen und dikotylen Pflanzen, insbesondere Gräsern, tropischen Bodenbedeckern und Tabakgeiztrieben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische der substituierte Naphthaline, Phthalsäureester wie Dibutyl- oder Dicotylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl-oder -äthyläther, Ketone wie Cyclohexanon, stark polare Lösungsmittel wie N-Methyl-2-pyrrolidon,Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier-und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyl-taurinsalzezu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Kaphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 10 Glykoläthergruppen und 8 bis 20 Kohlensotffatome im (aliphatischen) Kohlenwasserstoffrest und 6

bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffdatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthanol Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl-oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood, New Jersey, 1979.
Dr. Helmut Stache "Tensid Taschenbuch"
Carl Hanser Verlag, München/Wien 1981.

Die herbiziden Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent)

**Emulgierbare Konzentrate:**

| | | |
|---|---|---|
| Wirkstoff der Formel I: | 1 bis 20 % bevorzugt | 5 bis 10 % |
| oberflächenaktives Mittel: | 5 bis 30 %,vorzugsweise | 10 bis 20 % |
| flüssiges Trägermittel: | 50 bis 94 %,vorzugsweise | 70 bis 85 %. |

**Stäube:**

| | | |
|---|---|---|
| Wirkstoff der Formel I: | 0,1 bis 10 %, vorzugsweise | 0,1 bis 1 % |
| festes Trägermittel: | 99,9 bis 90 %, vorzugsweise | 99,9 bis 99 %. |

**Suspensions-Konzentrate:**

| | | |
|---|---|---|
| Wirkstoff der Formel I: | 5 bis 75 %, vorzugsweise | 10 bis 50 % |
| Wasser: | 94 bis 25 %, vorzugsweise | 90 bis 30 % |
| oberflächenaktives Mittel: | 1 bis 40 %, vorzugsweise | 2 bis 30 %. |

**Benetzbare Pulver:**

| | | |
|---|---|---|
| Wirkstoff der Formel I: | 0,5 bis 90 %, vorzugsweise | 1 bis 80 % |
| oberflächenaktives Mittel: | 0,5 bis 20 %, vorzugsweise | 1 bis 15 % |
| festes Trägermittel: | 5 bis 95 %, vorzugsweise | 15 bis 90 %. |

**Granulate:**

| | | |
|---|---|---|
| Wirkstoff der Formel I: | 0,5 bis 30 %, vorzugsweise | 3 bis 15 % |
| festes Trägermittel: | 99,5 bis 70 %, vorzugsweise | 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,005 bis 5 kg As/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele veranschaulichen die Herstellung einiger erfindungsgemässer Derivate des N-Phenyl-3,4,5,6-tetrahydrophthalsäureimides der Formel I. Weitere in entsprechender Weise hergestellte Verbindungen sind in den anschliessenden Tabellen aufgeführt. Temperaturangaben beziehen sich auf Celsius-Grade, Drucke sind in Millibar angegeben.

Beispiel 1

Herstellung von 2-Chlor-4-fluor-5-(N-3,4,5,6-tetrahydrophthalsäureimido)-acetophenon

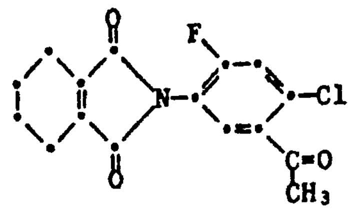

Ein Gemisch von 18,8 g 2-Chlor-4-fluor-5-aminoacetophenon, 15,2 g 3,4,5,6-Tetrahydrophthalsäureanhydrid und 300 ml Eisessig wird 8 Stunden unter Rühren am Rückfluss erhitzt. Danach wird das Reaktionsgemisch auf Eis gegossen und das ausgefallene Produkt mit Essigester extrahiert. Die organische Phase wird über Natriumsulfat getrocknet und dann unter Vakuum eingedampft. Der Rückstand wird in Methanol umkristallisiert. Man erhält 26,1 g 2-Chlor-4-fluor-5-(N-3,4,5,6-tetrahydrophthalsäureimido)-acetophenon mit einem Schmelzpunkt von 114° C.

Das als Ausgangsmaterial benötigte 2-Chlor-4-fluor-5-aminoacetophenon wird wie folgt hergestellt:

a) <u>2-Chlor-4-fluor-5-nitro-acetophenon</u>

Zu 500 ml rauchender Salpetersäure werden unter Rühren bei einer Temperatur von 0° tropfenweise 88 g 2-Chlor-4-fluor-acetophenon zugegeben. Nach zweistündigem Rühren bei dieser Temperatur wird das Reaktionsgemisch auf Eis gegossen, das ausgefallene Produkt abgenutscht und mit kaltem Aether gewaschen. Man erhält so 63 g 2-Chlor-4-fluor-5-nitroacetophenon mit einem Schmelzpunkt von 64-65° C.

b) <u>2-Chlor-4-fluor-5-amino-acetophenon</u>

In ein Gemisch von 97 g 2-Chlor-4-fluor-5-nitroacetophenon, 20 g Raney-Nickel und 1 Liter Tetrahydrofuran wird bei 20-25° C und unter normalem Druck Wasserstoff eingeleitet, bis die stöchiometrische Menge aufgenommen ist.

Nach Beendigung der Reaktion wird abfiltriert und das Filtrat unter Vakuum eingedampft. Der Rückstand wird aus Toluol/Cyclohexan kristallisiert und so erhält man 70 g 2-Chlor-4-fluor-5-aminoacetophenon mit einem Schmelzpunkt von 95-96° C.

In analoger Weise zu diesem Beispiel werden die 5-(N-3,4,5,6-Tetrahydrophthalsäureimido)-acetophenonderivate der Tabelle 1 hergestellt:

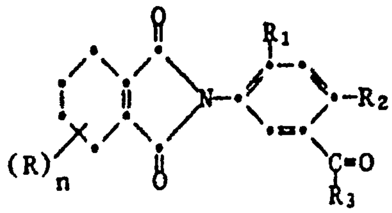

(Ia),

Tabelle 1

| No. | $(R)_n$ | $R_1$ | $R_2$ | $R_3$ | phys. Daten |
|---|---|---|---|---|---|
| 1.01 | -- | F | Cl | $CH_3$ | Smp. 114° |
| 1.02 | -- | F | H | $CH_3$ | Smp. 123-124° |
| 1.03 | -- | F | F | $CH_3$ | Smp. 124-125° |
| 1.05 | -- | F | Cl | $CH(CH_3)_2$ | |
| 1.06 | -- | F | Cl | $CH(CH_3)_2$ | |
| 1.07 | -- | F | F | $CH(CH_3)_2$ | |
| 1.09 | 4-$CH_3$ | F | Cl | $CH_3$ | |
| 1.10 | 4-$CH_3$ | F | H | $CH_3$ | |
| 1.11 | 4-$CH_3$ | F | F | $CH_3$ | |
| 1.13 | -- | F | Cl | $CF_3$ | |
| 1.14 | -- | F | H | $CF_3$ | |
| 1.15 | -- | F | F | $CF_3$ | |
| 1.17 | -- | F | Cl | $CH_2OCH_2$-$CH$=$CH_2$ | |
| 1.18 | -- | F | H | $CH_2OCH_2$-$CH$=$CH_2$ | |
| 1.19 | -- | F | F | $CH_2OCH_2$-$CH$=$CH_2$ | |
| 1.21 | 4-$CH_3$ | F | Cl | $CH_2O$-Benzyl | |
| 1.22 | 4-$CH_3$ | F | H | $CH_2O$-Benzyl | |
| 1.23 | 4-$CH_3$ | F | F | $CH_2O$-Benzyl | |
| 1.25 | -- | F | Cl | $CH_2OCH(CH_3)_2$ | |
| 1.26 | -- | F | H | $CH_2OCH(CH_3)_2$ | |
| 1.27 | -- | F | F | $CH_2OCH_2(CH_3)_2$ | |

Tabelle 1 (Fortsetzung)

| No. | $(R)_n$ | $R_1$ | $R_2$ | $R_3$ | phys. Daten |
|---|---|---|---|---|---|
| 1.29 | -- | F | Br | $CH_3$ | Smp. 124-5° |
| 1.30 | -- | F | Cl | $CH_2$-Br | Smp. 82° |
| 1.31 | 4-$CH_3$ | F | Br | $CH_3$ | |
| 1.32 | 3-$CH_3$ | F | Br | $CH_3$ | |

Beispiel 2

Herstellung von N-[2-Fluor-4-chlor-5-(2-methyl-1,3-dioxolan-2-yl)-phenyl]-3,4,5,6-tetrahydrophthalsäureimid

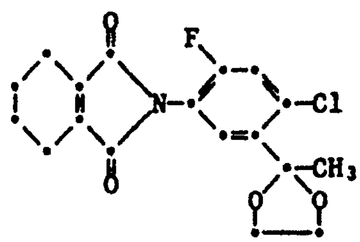

Ein Gemisch von 4,6 g 2-Chlor-4-fluor-5-(N-3,4,5,6-tetrahydrophthalsäureimido)-acetophenon, 1 g Aethylenglykol, 0,2 g p-Toluolsulfonsäure und 100 ml Benzol wird während 12 Stunden am Wasserabscheider gekocht. Das Reaktionsgemisch wird dann filtriert und die Lösung eingedampft. Der Rückstand wird aus Hexan umkristallisiert. Man erhält so 3,8 g Titelprodukt welches bei 147° schmilzt.

In analoger Weise zu diesem Beispiel werden die Acetalderivate des 5-(N-3,4,5,6-Tetrahydrophthalsäureimido)-phenons der Tabelle 2 hergestellt:

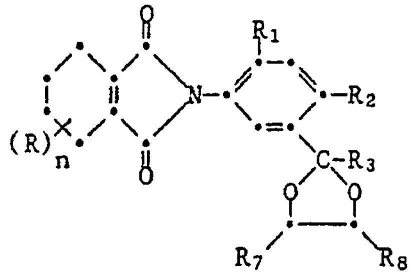

Tabelle 2

| No. | (R)n | R1 | R2 | R3 | R7 | R8 | phys. Daten |
|---|---|---|---|---|---|---|---|
| 2.01 | -- | F | Cl | $CH_3$ | H | H | Smp. 147° |
| 2.02 | -- | F | Cl | $CH_3$ | $CH_3$ | H | Smp. 78° |
| 2.03 | -- | F | Cl | $CH_3$ | $C_2H_5$ | H | $n_D^{30}$ 1,5227 |
| 2.04 | -- | F | Cl | $CH_3$ | $C_3H_7n$ | H | |
| 2.05 | 3-$CH_3$ | F | Cl | $CH_3$ | $CH(CH_3)_2$ | H | |
| 2.06 | -- | F | Cl | $CH_3$ | $C_4H_9n$ | H | |
| 2.07 | 3,4-$(CH_3)_2$ | F | Cl | $CH_3$ | $CH_2CH(CH_3)_2$ | H | |
| 2.08 | -- | F | Cl | $CH_3$ | $CH_2Cl$ | H | $n_D^{26}$ 1,5401 |
| 2.09 | -- | F | Cl | $CH_3$ | $CH_2OH$ | H | $n_D^{26}$ 1,5513 |
| 2.10 | -- | F | Cl | $CH_3$ | $CH_2O$-(4-chlorbenzyl) | H | |
| 2.11 | -- | F | Cl | $CH_3$ | $CH_2O$-(4-methylbenzyl) | H | |
| 2.12 | -- | F | Cl | $CH_3$ | $CH_2OCH_3$ | H | |
| 2.13 | -- | F | Cl | $CH_3$ | $CH_2OC_2H_5$ | H | |
| 2.14 | -- | F | Cl | $CH_3$ | $CH_2OCH_2CH=CH_2$ | H | |
| 2.15 | -- | F | Cl | $CH_3$ | $CH_2OCH_2C\equiv CH$ | H | |
| 2.16 | -- | F | Cl | $CH_3$ | $CH_3$ | $CH_3$ | $n_D^{27}$ 1,5252 |
| 2.17 | -- | F | Cl | $CH_3$ | $CH_3$ | $C_2H_5$ | |
| 2.18 | -- | F | Cl | $CH_3$ | $CH_3$ | $C_3H_7n$ | |
| 2.19 | 3-$CH_3$ | F | H | $CH_3$ | $CH_3$ | $CH(CH_3)_2$ | |
| 2.20 | -- | F | Cl | $CH_3$ | $C_2H_5$ | $CH_3$ | |
| 2.21 | -- | F | Cl | $CH_3$ | $C_2H_5$ | $C_2H_5$ | |
| 2.22 | -- | F | Cl | $CH_3$ | $C_2H_5$ | $C_3H_7n$ | |

Tabelle 2 (Fortsetzung)

| No. | $(R)_n$ | $R_1$ | $R_2$ | $R_3$ | $R_7$ | $R_8$ | phys. Daten |
|---|---|---|---|---|---|---|---|
| 2.23 | 3,4-$(CH_3)_2$ | F | Cl | $CH_3$ | $C_2H_5$ | $CH(CH_3)_2$ | |
| 2.24 | -- | F | H | $CH_3$ | H | H | Smp.160° |
| 2.25 | -- | F | H | $CH_3$ | $CH_3$ | $CH_3$ | Harz |
| 2.26 | -- | F | H | $CH_3$ | $C_2H_5$ | $CH_3$ | |
| 2.27 | -- | F | H | $CH_3$ | $C_2H_5$ | $C_2H_5$ | |
| 2.28 | 3-$(CH_3)$ | F | H | $CH_3$ | $C_2H_5$ | $CH(CH_3)_2$ | |
| 2.29 | -- | F | H | $CH_3$ | $C_2H_5$ | $C_3H_7n$ | |
| 2.30 | -- | F | Cl | $CH_3$ | $CH_2OCH_2C{\equiv}CH$ | H | |
| 2.31 | -- | F | F | $CH_3$ | $CH_3$ | H | $n_D^{26}$ 1,5347 |
| 2.32 | -- | F | F | $CH_3$ | $CH_3$ | $CH_3$ | Harz |
| 2.33 | -- | F | H | $CH_3$ | $C_2H_5$ | H | Smp.65-67° |
| 2.34 | -- | F | Cl | $-CH_2Br$ | $CH_3$ | H | $n_D^{23}$ 1,5410 |
| 2.35 | -- | F | H | $CH_3$ | $CH_3$ | H | $n_D^{26}$ 1,5347 |
| 2.36 | -- | F | H | $CH_3$ | $C_3H_7$-n | H | $n_D^{26}$ 1,5193 |
| 2.37 | 3-$CH_3$ | F | Br | $CH_3$ | $CH_3$ | $CH_3$ | |
| 2.38 | 4-$CH_3$ | F | Br | $CH_3$ | $CH_3$ | $CH_3$ | |
| 2.39 | -- | F | Br | $CH_3$ | H | H | |
| 2.40 | -- | F | Br | $CH_3$ | $CH_3$ | H | |
| 2.41 | -- | F | Br | $CH_3$ | $C_2H_5$ | H | |
| 2.42 | -- | F | Br | $CH_3$ | $C_3H_7$ | H | |
| 2.43 | -- | F | Br | $CH_3$ | $CH(CH_3)_2$ | H | |
| 2.44 | -- | F | Br | $CH_3$ | $C_4H_9(n)$ | H | |
| 2.45 | -- | F | Br | $CH_3$ | $CH_2Cl$ | H | |
| 2.46 | -- | F | Br | $CH_3$ | $CH_2OH$ | H | |
| 2.47 | -- | F | Br | $CH_3$ | $CH_3$ | $CH_3$ | |
| 2.48 | -- | F | Br | $CH_3$ | $CH_3$ | $C_2H_5$ | |
| 2.49 | 3-$CH_3$ | F | Cl | $CH_3$ | $CH_3$ | $CH_3$ | |
| 2.50 | 4-$CH_3$ | F | Cl | $CH_3$ | $CH_3$ | $CH_3$ | |

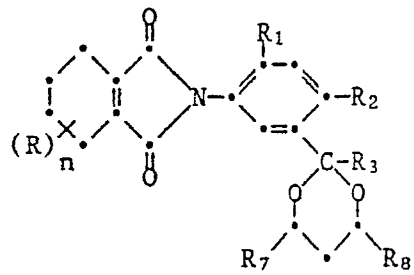

Tabelle 2A

| No. | $(R)_n$ | $R_1$ | $R_2$ | $R_3$ | $R_7$ | $R_8$ | phys. Daten |
|---|---|---|---|---|---|---|---|
| 2.51 | -- | F | H | $CH_3$ | H | $CH_3$ | Smp.132° |
| 2.52 | -- | F | Cl | $CH_3$ | H | H | Smp.152-153° |

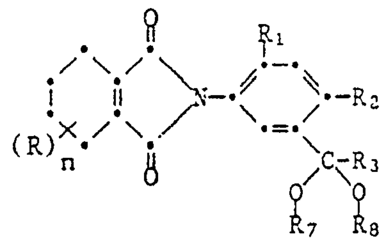

Tabelle 2B

| No. | $(R)_n$ | $R_1$ | $R_2$ | $R_3$ | $R_7$ | $R_8$ |
|---|---|---|---|---|---|---|
| 2.53 | -- | F | Cl | $CH_3$ | $CH_3$ | $CH_3$ |
| 2.54 | -- | F | Cl | $CH_3$ | $C_2H_5$ | $C_2H_5$ |
| 2.55 | -- | F | Cl | $CH_3$ | $C_3H_7$ | $C_3H_7$ |
| 2.56 | -- | F | Cl | $CH_3$ | $CH(CH_3)_2$ | $CH(CH_3)_2$ |
| 2.57 | -- | F | Cl | $CH_3$ | $-C_4H_9$-n | $-C_4H_9$-n |
| 2.58 | -- | F | Br | $CH_3$ | $-CH_3$ | $-CH_3$ |
| 2.59 | -- | F | Br | $CH_3$ | $C_2H_5$ | $C_2H_5$ |
| 2.60 | -- | F | Br | $CH_3$ | $C_3H_7$-n | $C_3H_7$-n |
| 2.61 | -- | F | Br | $CH_3$ | $CH(CH_3)_2$ | $CH(CH_3)_2$ |
| 2.62 | -- | F | Br | $CH_3$ | $-C_4H_9$-n | $C_4H_9$-n |
| 2.63 | 4-$CH_3$ | F | Cl | $CH_3$ | $CH_3$ | $CH_3$ |
| 2.64 | 3-$CH_3$ | F | Cl | $CH_3$ | $CH_3$ | $CH_3$ |

Beispiel 3

Herstellung von 2-Chlor-4-fluor-5-(N-3,4,5,6-tetrahydropthalsäureimido)-acetophenon Oxim

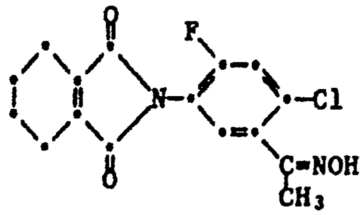

Ein Gemisch von 3,2 g 2-Chlor-4-fluor-5-(N-3,4,5,6-tetrahydropthalsäureimido)-acetophenon, 0,8 g Hydroxylamin • Hydrochlorid und 50 ml Aethanol wird während 2 Stunden unter Rühren am Rückfluss erhitzt. Nach Beendigung der Reaktion wird die Lösung eingedampft, der Rückstand in Methanol aufgenommen, mit Aktivkohle gereinigt und filtriert. Das Filtrat wird erneut eingedampft und aus dem Rückstand kristallisiert das 2-Chlor-4-fluor-5-(N-3,4,5,6-tetrahydrophtalsäureimido)acetophenonoxim aus. Die Ausbeute beträgt 2,5 g. Schmelzpunkt 179 ° C.

In analoger Weise zu diesem Beispiel werden die 5-(N-3,4,5,6-tetrahydro-phthalsäure)-acetophenonoxime der Tabellen 3 und 3A hergestellt:

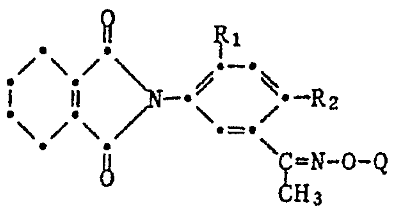

Tabelle 3

| No. | $R_1$ | $R_2$ | Q | phys. Daten |
|---|---|---|---|---|
| 3.01 | F | Cl | H | Smp. 179° |
| 3.02 | F | H | H | Smp. 186° |
| 3.03 | F | F | H | Smp. 142° |
| 3.04 | F | Cl | $CH_3$ | $n_D^{27}$ 1.5479 |
| 3.05 | F | Cl | $C_2H_5$ | $n_D^{22}$ 1.5469 |
| 3.06 | F | Cl | $C_3H_7n$ | |
| 3.07 | F | Cl | $CH(CH_3)_2$ | |
| 3.08 | F | Cl | $CH_2CN$ | |
| 3.09 | F | Cl | $C_2H_4OCH_3$ | |
| 3.10 | F | Cl | $CH_2CH{=}CH_2$ | $n_D^{26}$ 1.5528 |
| 3.11 | F | Cl | $CH_2CCl{=}CH_2$ | |
| 3.12 | F | Cl | $CH_2CH{=}CHCl$ | $n_D^{26}$ 1.5158 |
| 3.13 | F | Cl | $CH_2C{\equiv}CH$ | |
| 3.14 | F | Cl | Hexyl | $n_D^{26}$ 1.5683 Harz |

Tabelle 3 (Fortsetzung)

| No. | $R_1$ | $R_2$ | Q | phys. Daten |
|---|---|---|---|---|
| 3.16 | F | Cl | Benzoyl | |
| 3.17 | F | Cl | $SO_2CH_3$ | |
| 3.18 | F | Cl | $SO_2N(CH_3)_2$ | |
| 3.19 | F | Cl | $COOCH_3$ | |
| 3.20 | F | Cl | $COOC_2H_5$ | |
| 3.21 | F | Cl | $COSCH_3$ | |
| 3.22 | F | Cl | $COSC_2H_5$ | |
| 3.23 | F | Cl | $CONHCH_3$ | |
| 3.24 | F | Cl | Benzyl | Smp. 130-132° |
| 3.25 | F | Cl | 4-Chlorbenzyl | |
| 3.26 | F | Cl | 2-Chlorbenzyl | $n_D^{28}$ 1.5672 |
| 3.27 | F | Cl | 4-Methylbenzyl | |
| 3.28 | F | Cl | 2-Furylcarbonyl | |
| 3.29 | F | Cl | 2-Thienylcarbonyl | |
| 3.30 | F | Cl | $C_4H_9n$ | $n_D^{26}$ 1.5469 |
| 3.31 | F | Cl | $C(CH_3)_3$ | $n_D^{26}$ 1.5159 |
| 3.32 | F | F | 2-Chlorbenzyl | Smp. 93-94° |
| 3.33 | F | F | Benzyl | Smp.107-109° |
| 3.34 | F | Cl | $(CH_2)_5CH_3$ | $n_D^{26}$ 1.5383 |

Tabelle 3 (Fortsetzung)

| No. | $R_1$ | $R_2$ | Q | phys. Daten |
|---|---|---|---|---|
| 3.35 | F | Cl | $CH(CH_3)-CN$ | |
| 3.36 | F | Br | H | |
| 3.37 | F | Br | $-CH_3$ | |
| 3.38 | F | Br | $C_2H_5$ | |
| 3.39 | F | Br | $-C_3H_7-n$ | |
| 3.40 | F | Br | $CH(CH_3)_2$ | |
| 3.41 | F | Br | $-CH_2-CN$ | |
| 3.43 | F | Br | $CH_2-CH=CH_2$ | |
| 3.44 | F | Br | $CH_2-C(Cl)=CH_2$ | |
| 3.45 | F | Br | $-CH_2-CH=CHCl$ | |
| 3.46 | F | Br | $-CH_2-C\equiv CH$ | |
| 3.47 | F | Br | Cyclohexyl | |
| 3.49 | F | Br | Benzyl | |

Tabelle 3 (Fortsetzung)

| No. | $R_1$ | $R_2$ | Q | phys. Daten |
|---|---|---|---|---|
| 3.50 | F | Br | $SO_2-CH_3$ | |
| 3.51 | F | Br | $SO_2-N(CH_3)_2$ | |
| 3.52 | F | Br | $-COOCH_3$ | |
| 3.53 | F | Br | $-COOC_2H_5$ | |
| 3.54 | F | Br | $-COSCH_3$ | |
| 3.55 | F | Br | $-COSC_2H_5$ | |
| 3.56 | F | Br | $CONHCH_3$ | |
| 3.100 | F | Br | Benzyl | |

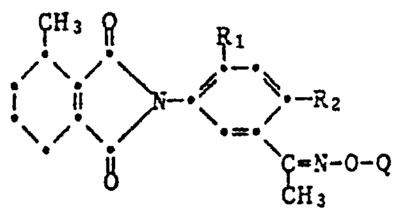

Tabelle 3A

| No. | $R_1$ | $R_2$ | Q | phys. Daten |
|---|---|---|---|---|
| 3.101 | F | Cl | H | |
| 3.102 | F | Cl | $CH_3$ | |
| 3.103 | F | Cl | $C_2H_5$ | |

Beispiel 4:

Herstellung von Methoximino-[2-chlor-4-fluor-5-(N-3,4,5,6-tetrahydrophthalsäureimido)-benzoylcyanid (2-Chlor-4-fluor-5-(N-3,4,5,6-tetrahydrophthalsäureimido)-phenylglyoxylonitril-2-oximinomethyl-äther)

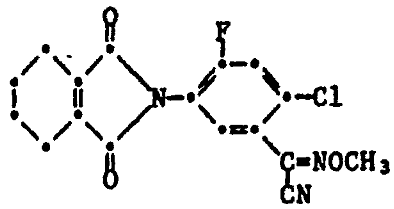

Man rührt bei Rückflusstemperatur während 12 Stunden ein Gemisch von 8,5 g 5-Amino-2-chlor-4-fluorphenylglyoxylonitril-2-oximinoäther, 3 g 3,4,5,6-Tetrahydrophthalsäureanhydrid und 100 ml Propionsäure. Dann wird das Reaktionsgemisch unter Vakuum eingedampft. Der Rückstand wird in Toluol/Hexan aufgenommen und zum Kristallisieren gebracht. Man erhält so 8,5 g Titelprodukt welches bei 97-99 °C schmilzt.

Der als Ausgangsprodukt benötigte 5-Amino-2-chlor-4-fluorphenylglyoxylonitril-2-oximinoäther wird wie folgt hergestellt:

a) Na-Salz des Oxims vom 2-Chlor-4-fluor-benzoyl-cyanid (2-Chlor-4-fluorphenylglyoxylonitril-2-oxim-Natriumsalz)

In einem Dreihalskolben werden unter Rühren bei Stickstoffatmosphäre 9,2 g Natrium in 300 ml Aethanol gelöst. Dann gibt man tropfenweise bei Raumtemperatur zuerst 67,7 g 2-Chlor-4-fluor-benzylcyanid und anschliessend 48 g Isopentylnitrit dazu. Es entsteht eine gelbe Suspension, die man 4 Stunden bei Raumtemperatur weiterrührt und dann einengt. Der Rückstand wird in Methylenchlorid aufgenommen verrührt und filtriert. Der Filterrückstand ist das gewünschte Natrium-Salz. Ausbeute 82 g, Smp. 287 °C.

Das freie Oxim wird durch Verrühren in verdünnter Salzsäure erhalten. Smp. 129 °C.

b) Methoximino-2-chlor-4-fluorbenzoylcyanid (2-Chlor-4-fluor-phenylglyoxylonitril-2-oximinomethyläther)

Man rührt bei 60 °C während 12 Stunden ein Gemisch aus 40 g Na-Salz des Oxims vom 2-Chlor-4-fluor-benzoylcyanids (2-Chlor-4-fluorphenylglyoxylonitril-2-oxim) 30 g Methyljodid und 500 ml Methyläthylketon. Das Reaktionsgemisch wird dann filtriert und das Filtrat eingedampft. Man erhält so 24 g Titelprodukt welches bei 66-67° schmilzt.

c) Methoximino-2-chlor-4-fluor-5-nitrobenzoylcyanid (2-Chlor-4-fluor-5-nitro-phenylglyoxylonitril-2-oximinomethyläther)

Man gibt vorsichtig in kleinen Portionen unter Rühren bei 0 °C 21,2 g 2-Chlor-4-fluor-phenyloxylonitril-2-oximinomethyläther in 200 ml konzentrierte Schwefelsäure, wobei eine gelbe Lösung entsteht. Dazu tropft man langsam 4,5 ml rauchende Salpetersäure. Nachdem alles zugetropft ist lässt man die Lösung sich auf Raumtemperatur erwärmen und rührt noch 3 Stunden lang weiter. Dann wird das Reaktionsgemisch auf Eis gegossen und der ausgefallene Niederschlag abgenutscht und getrocknet. Man erhält so 23 g Titelprodukt, welches bei 85-86 °C schmilzt.

Methoximino-5-amino-2-chlor-4-fluor-benzoylcyanid (5-Amino-2-chlor-4-fluor-phenylglyoxylonitril-2-oximinomethyläther)

Ein Gemisch von 12 g 2-Chlor-4-fluor-5-nitrophenylglyoxylnitril-2-oximinomethyläther, 37,2 g Zinndichlorid-Dihydrat und 43 ml konzentrierte Salzsäure wird auf 100 °C erwärmt und bei dieser Temperatur während 10 Minuten weitergerüht. Dann giesst man das Reaktionsgemisch auf Eis, stellt mit Natriumbicarbonatlösung alkalisch und extrahiert das organische Material mit Essigsäureäthylester. Die organische Phase wird über Natriumsulfat getrocknet, filtriert und eingedampft. Der Rückstand ist das gewünschte Amin. Ausbeute 9 g Schmelzpunkt 71 °C.

In analoger Weise zu diesem Beispiel werden die in den Tabellen 4, 4A und 4B aufgeführten 5-(N-3,4,5,6-Tetrahydrophthalsäureimido)-benzoylcyanid-oximähter hergestellt.

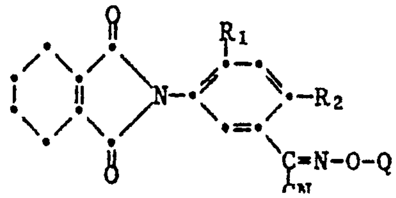

Tabelle 4

| No. | $R_1$ | $R_2$ | Q | phys. Daten |
|---|---|---|---|---|
| 4.01 | F | Cl | H | |
| 4.02 | F | F | H | |
| 4.03 | F | H | H | |
| 4.04 | F | Cl | $CH_3$ | Smp. 97-99° |
| 4.05 | F | Cl | $C_2H_5$ | Smp. 125° |
| 4.06 | F | Cl | $C_3H_7n$ | |
| 4.07 | F | Cl | $CH(CH_3)_2$ | Smp. 98-99° |
| 4.08 | F | Cl | $CH_2CH{=}CH_2$ | |
| 4.09 | F | Cl | $CH_2CCl{=}CH_2$ | |
| 4.10 | F | Cl | $CH_2C{\equiv}CH$ | |
| 4.11 | F | Cl | Benzoyl | |
| 4.12 | F | Cl | 4-Chlorbenzoyl | |

Tabelle 4

| No. | $R_1$ | $R_2$ | Q | phys. Daten |
|---|---|---|---|---|
| 4.13 | F | Cl | $COOCH_3$ | |
| 4.14 | F | Cl | $COOC_2H_5$ | |
| 4.15 | F | Cl | $CONHCH_3$ | |
| 4.16 | F | Cl | $CON(CH_3)_2$ | |
| 4.17 | F | Cl | Benzyl | |
| 4.18 | F | Cl | Cyclohexyl | |
| 4.19 | F | Cl | 2-Chlorbenzyl | |
| 4.20 | F | Cl | 4-Methylbenzyl | |
| 4.21 | F | Cl | 2-Furylcarbonyl | |
| 4.22 | F | Cl | 2-Thienylcarbonyl | |
| 4.23 | F | Cl | $CH(CH_3)C_2H_5$ | Smp. 106-107° |
| 4.24 | F | Cl | $CH_2CH(CH_3)_2$ | |
| 4.25 | F | Cl | $C(CH_3)_3$ | |
| 4.26 | F | Cl | $C_5H_{11}n$ | |
| 4.27 | F | Cl | $CH_2CN$ | |
| 4.28 | F | Cl | $CH_2CH_2Cl$ | |
| 4.29 | F | Cl | $SO_2CH_3$ | |
| 4.30 | F | Cl | $SO_2N(CH_3)_2$ | |
| 4.31 | F | Cl | $COSCH_3$ | |

Tabelle 4 (Fortsetzung)

| No. | $R_1$ | $R_2$ | Q | phys. Daten |
|---|---|---|---|---|
| 4.32 | F | Cl | $C_4H_9(n)$ | |
| 4.41 | F | Cl | $CH(CH_3)CN$ | |
| 4.44 | F | Cl | $CO-SC_2H_5$ | |
| 4.50 | F | Cl | $-CH_2-C(CH_3)_3$ | |
| 4.51 | F | Cl | $-CH_2-CH=CH_2$ | |
| 4.52 | F | Cl | $-CH_2-C\equiv CH$ | |
| 4.53 | F | Br | H | |
| 4.54 | F | Br | $CH_3$ | |
| 4.55 | F | Br | $C_2H_5$ | |
| 4.56 | F | Br | $C_3H_7-n$ | |
| 4.57 | F | Br | $CH(CH_3)_2$ | |
| 4.58 | F | Br | $C_4H_9-n$ | |
| 4.59 | F | Br | $CH_2-CH(CH_3)_2$ | |
| 4.60 | F | Br | $CH(CH_3)-CH_2-CH_3$ | |
| 4.61 | F | Br | $C_5H_{11}-n$ | |
| 4.62 | F | Br | $CH_2-CH=CH_2$ | |
| 4.63 | F | Br | $CH_2-\underset{Cl}{C}=CH_2$ | |
| 4.64 | F | Br | $CH_2-CH=CHCl$ | |
| 4.65 | F | Br | $CH_2-CH=CCl-CH_3$ | |

Tabelle 4 (Fortsetzung)

| No. | $R_1$ | $R_2$ | Q | phys. Daten |
|---|---|---|---|---|
| 4.66 | F | Br | $CH_2-C\equiv CH$ | |
| 4.67 | F | Br | Benzoyl | |
| 4.68 | F | Br | $COOCH_3$ | |
| 4.69 | F | Br | $COOC_2H_5$ | |
| 4.70 | F | Br | $COSCH_3$ | |
| 4.71 | F | Br | $COSC_2H_5$ | |
| 4.72 | F | Br | $CONHCH_3$ | |
| 4.73 | F | Br | Benzyl | |
| 4.74 | F | Br | $CH_2-CN$ | |
| 4.75 | F | Br | $CH(CH_3)-CN$ | |
| 4.76 | F | Br | $CH_2-CH_2-Cl$ | |
| 4.77 | F | Br | $SO_2-CH_3$ | |

Tabelle 4 (Fortsetzung)

| No. | $R_1$ | $R_2$ | Q | phys. Daten |
|---|---|---|---|---|
| 4.78 | F | Br | $SO_2-N(CH_3)_2$ | |
| 4.79 | F | Cl | $CH_2-C(CH_3)=CH_2$ | |
| 4.80 | F | Cl | $CH_2-C(CH_3)=CH_2$ | |

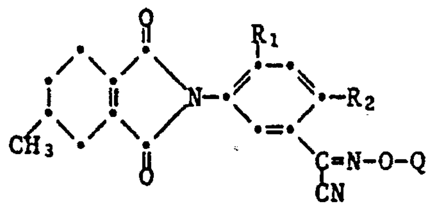

Tabelle 4A

| No. | $R_1$ | $R_2$ | Q | phys. Daten |
|---|---|---|---|---|
| 4.151 | F | Br | H | |
| 4.152 | F | Br | $CH_3$ | |
| 4.153 | F | Br | $C_2H_5$ | |
| 4.154 | F | Br | $CH(CH_3)_2$ | |
| 4.155 | F | Br | $CH_2-CH=CH_2$ | |
| 4.156 | F | Br | $CH_2-CH=CHCl$ | |
| 4.157 | F | Br | $CH_2-C\equiv CH$ | |

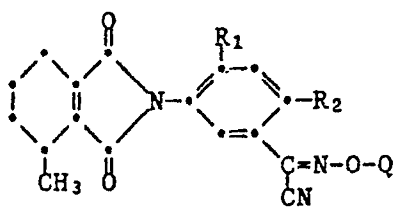

Tabelle 4B (Fortsetzung)

| No. | $R_1$ | $R_2$ | Q | phys. Daten |
|---|---|---|---|---|
| 4.158 | F | Cl | H | |
| 4.159 | F | Cl | $CH_3$ | |
| 4.160 | F | Cl | $C_2H_5$ | |
| 4.161 | F | Cl | $CH(CH_3)_2$ | |
| 4.162 | F | Cl | $CH_2-CH=CH_2$ | |
| 4.163 | F | Cl | $CH_2-C(CH_3)=CH_2$ | |
| 4.164 | F | Cl | $CH_2-CH=CHCl$ | |
| 4.165 | F | Cl | $CH_2-C\equiv CH$ | |

Formulierungsbeispiele:

Beispiel 6: Formulierungsbeispiele für Wirkstoffe der Formel I (% = Gewichtsprozent)

| a) Spritzpulver | a) | b) | c) |
|---|---|---|---|
| Wirkstoff gemäss Tabelle 1 oder 2 | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther (7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff gemäss Tabelle 1 oder 2 | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff gemäss Tabelle 1 oder 2 | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff gemäss Tabelle 1 oder 2 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungs-Granulat | |
|---|---|
| Wirkstoff gemäss Tabelle 1 oder 2 | 3 % |
| Polyäthylenglykol (MG 200) | 3 % |
| Kaolin | 94 % |

Der fein gemahlene Wirkstoff wird in eines Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff gemäss Tabelle 1 oder 2 | 40 % | 5 % |
| Aethylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

| g) Salzlösung | |
|---|---|
| Wirkstoff gemäss Tabelle 1 oder 2 | 5 % |
| Isopropylamin | 1 % |
| Octylphenolpolyäthylenglykoläther (78 Mol AeO) | 3 % |
| Wasser | 91 % |

Biologische Beispiele:

Beispiel 7: Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25 %igen Emulsionskonzentrat behandelt. Es werden Konzentrationen von 4 kg Wirksubstanz/Hektar angewendet. Die Saatschalen werden im Gewächshaus bei 22-25 °C und 50-70 % relativer Luftfeuchtigkeit gehalten und der Versuch nach 3 Wochen ausgewertet.

In diesem Versuch zeigen die Verbindungen der Tabellen 1 und 2 starke Herbizidwirkung.

Beispiel 8: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wässrigen Wirkstoffdispersion in einer Dosierung von 4 kg Wirksubstanz pro Hektar auf die Pflanzen gespritzt und diese bei 24 °-26 °C und 45-60 % relativer Luftfeuchtigkeit gehalten. 15 Tage nach der Behandlung wird der Versuch ausgewertet. Auch in diesem Versuch zeigen die Verbindungen der Tabellen 1 und 2 starke bis sehr starke Herbizidwirkung.

Beispiel 9: Herbizidwirkung vor dem Auflaufen der Pflanzen

Kunststofftöpfe werden mit expandiertem Vermiculit (Dichte: 0,135 g/cm$^3$, Wasserabsorptionsvermögen: 0,565 1/1) gefüllt. Nach dem Sättigen des nicht adsorptiven Vermiculits mit einer wässrigen Wirkstoffemulsion in deinonisiertem Wasser, die die Wirkstoffe in einer Konzentration von 70,8 ppm enthält, werden Samen der folgenden Pflanzen auf die Oberfläche gesät. Nasturtium offcinalis, Agrostis tenuis, Stellaria media und Digitaria sanguinalis. Die Versuchsgefässe werden anschliessend in einer Klimakammer bei 20 °C, einer Beleuchtung von ca. 20 kLux und einer relativen Luftfeuchtigkeit von 70 % gehalten. Während der Keimphase von 4 bis 6 Tagen werden die Töpfe zur Erhöhung der örtlichen Luftfeuchtigkeit mit lichtdurchlässigem Material abgedeckt und mit deinoisiertem Wasser begossen. Nach dem 5. Tag wird dem Giesswasser 0,5 % eines handelsüblichen Flüssigdüngers (®Greenzit) zugesetzt. 12 Tage nach der Aussaat wird der Versuch ausgewertet und die Wirkung auf die Versuchspflanzen bewertet.

Der Zustand der Pflanzen wird dabei gemäss den folgenden Bewertungsskalen bonitiert:

1 Pflanze abgestorben oder hat nicht gekeimt.

2-8 linear abnehmende Schadstufen

9 keine Schädigung die Pflanze gedeiht wie unbehandelte Kontrollpflanzen

Die Resultate sind in der Tabelle 5 zusammengestellt:

Tabelle 5

| Verbindung | Nasturtium | Stellaria | Agrostis | Digitaria |
|---|---|---|---|---|
| 1.01 | 1 | 1 | 1 | 1 |
| 1.02 | 2 | 2 | 2 | 2 |
| 1.03 | 1 | 1 | 1 | 1 |
| 1.22 | 1 | 1 | 1 | 1 |
| 1.23 | 1 | 1 | 1 | 1 |
| 2.01 | 1 | 1 | 1 | 1 |
| 2.02 | 1 | 1 | 1 | 1 |
| 2.03 | 1 | 1 | 1 | 1 |
| 2.08 | 1 | 1 | 1 | 1 |
| 2.09 | 1 | 1 | 1 | 1 |
| 2.16 | 1 | 1 | 1 | 1 |
| 2.24 | 3 | 5 | 4 | 3 |
| 2.25 | 6 | 6 | 4 | 6 |
| 2.33 | 6 | 4 | 4 | 6 |
| 2.35 | 3 | 4 | 2 | 2 |
| 2.37 | 3 | 3 | 3 | 3 |
| 2.36 | 1 | 1 | 1 | 1 |
| 2.40 | 1 | 1 | 1 | 1 |
| 3.01 | 1 | 1 | 1 | 1 |
| 3.02 | 2 | 2 | 2 | 2 |
| 3.04 | 1 | 1 | 1 | 1 |
| 3.05 | 1 | 1 | 1 | 1 |
| 3.10 | 1 | 1 | 1 | 1 |

Tabelle 5 (Fortsetzung)

| Verbindung | Nasturtium | Stellaria | Agrostis | Digitaria |
|---|---|---|---|---|
| 3.22 | 3 | 3 | 3 | 3 |
| 3.24 | 1 | 1 | 1 | 1 |
| 3.26 | 2 | 2 | 1 | 1 |
| 3.30 | 1 | 1 | 1 | 1 |
| 3.31 | 1 | 1 | 1 | 1 |
| 3.32 | 2 | 2 | 2 | 2 |
| 3.33 | 2 | 2 | 2 | 2 |
| 3.34 | 1 | 1 | 1 | 1 |
| 4.04 | 1 | 1 | 1 | 1 |
| 4.05 | 1 | 1 | 1 | 1 |
| 4.06 | 1 | 1 | 1 | 1 |

Beispiel 10 Herbizidwirkung für Wasserreis (paddy)

Die Wasserunkräuter Echinochloa crus galli und Monocharia vag. werden in Plastikbechern (60 $cm^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat mit einer wässrigen Emulsion der Prüfsubstanzen durch eine Spritzung auf die Gefässe. Die daher verwendete Dosis entspricht einer Wirkstoffmenge von 0,5 bis 4 kg AS pro Hektar (Spritzbrühmenge = 550 1/ha. Die Pflanzenbecher werden dann im Gewächshaus unter optimalen Wachstumsbedingungen für die Reisunkräuter aufgestellt, d.h. bei 25°-30°C und hoher Luftfeuchtigkeit.

Die Auswertung der Versuche findet 3 Wochen nach Applikation statt. Die Verbindungen der Tabellen 1 und 2 schädigen dabei die Unkräuter, nicht aber den Reis.

Beispiel 11: Wuchshemmung bei tropischen Bodenbedecker-Leguminosen (cover crops).

Die Versuchspflanzen (centrosema plumieri und centrosema pubescens) werden bis zum ausgewachsenen Stadium herangezogen und bis auf eine Höhe von 60 cm zurückgeschnitten. Nach 7 Tagen wird der Wirkstoff als wässrige Emulsion gespritzt. Die Versuchspflanzen werden bei 70 % relativer Luftfeuchtigkeit und 6000 lux Kunstlicht, pro Tag 14 Stunden, bei Temperaturen von 27° bei Tag und 21°C bei Nacht gehalten. 4 Wochen nach der Applikation wird der Versuch ausgewertet. Es werden dabei der Neuzuwachs im Vergleich zur Kontrolle abgeschätzt und gewogen und die Phytotoxizität bewertet. In diesem Versuch zeigen die mit den Wirkstoffen der Tabellen 1 und 2 in Aufwandmengen von 50-3000 g/ha behandelten Pflanzen eine deutliche Reduktion des Neuzuwachses (weniger als 20 % des Neuzuwachses bei unbehandelten Kontrollpflanzen), ohne dass dabei die Versuchspflanzen geschädigt wurden.

Beispiel 12: Wuchsregulierung an Sojabohnen

In Kunststoffbehältern mit einem Erde-Torf-Sandgemisch im Verhältnis 6:3:1 werden Sojabohnen der Sorte "Hark" angesät und in eine Klimakammer gegeben. Durch optimale Tempeaturwahl, Beleuchtung, Düngerzugabe und Bewässerung entwickeln sich die Pflanzen nach ca. 5 Wochen bis zum 5-6 Trifolia-Blattstadium. Zu diesem Zeitpunkt werden die Pflanzen mit der wässrigen Brühe eines Wirkstoffs der Formel I bis zur guten Benetzung besprüht. Die Wirkstoffkonzentration beträgt bis zu 100 g AS/ha. Die

Auswertung erfolgt ca. 5 Wochen nach der Applikation des Wirkstoffs. Im Vergleich zu unbehandelten Kontrollpflanzen bewirken die erfindungsgemässen Wirkstoffe der Tabellen 1 und 2 eine merklicher Erhöhung der Anzahl und des Gewichte der Schoten im Haupttrieb.

Beispiel 13: Wuchshemmung bei Getreide

In Kunststofftöpfen mit sterilisierter Erde werden die Getreidearten Hordeum vulgare (Sommergerste) und Secale (Sommerroggen) im Gewächshaus angesät und nach Bedarf bewässert. Die Sprösslinge werden ca. 21 Tage nach der Aussaat mit der wässrigen Spritzbrühe eines Wirkstoffes der Tabelle 1 oder 2 besprüht. Die Wirkstoffmenge beträgt bis zu 100 g Aktivsubstanz pro Hektar. 21 Tage nach Applikation wird das Wachstum des Getreides beurteilt. Die behandelten Pflanzen weisen im Vergleich zu unbehandelten Kontrollen eine Verringerung des Neuzuwachses (60-90 % der Kontrolle), sowie teilweise eine Zunahme der Stengeldurchmesser auf.

Beispiel 14: Wuchshemmung bei Gräsern

In Kunststoffschalen mit Erde-Torf-Sand-Gemisch (6:3:1) werden die Gräser Lolium perenne, Poa pratensis, Festuca ovina, Dactylis glomerate und Cynodon dactylon im Gewächshaus angesät und nach Bedarf bewässert. Die aufgeleufenen Gräser werden wöchentlich bis auf 4 cm Höhe zurückgeschnitten und ca. 50 Tage nach der Aussaat und einen Tag nach dem letzten Schnitt mit der wässrigen Spritzbrühe eines Wirkstoffes der Tabellen 1 und 2 besprüht. Die Wirkstoffmenge beträgt umgerechnet bis zu 500 g Aktivsubstanz pro Hektar. 21 Tage nach Applikation wird das Wachstum der Gräser beurteilt.

Die geprüften Verbindungen der Tabellen 1 und 2 bewirken eine Reduzierung des Neuzuwachses von um 10-30 % im Vergleich zur unbehandelten Kontrolle.

Beispiel 15: Desiccations- und Defoliationswirkung

In einem Gewächshaus wurden Baumwollpflanzen der Sorte Deltapine in Tontöpfen angezogen. Nach abgeschlossener Kapselbildung wurden sie mit wässerigen Zubereitungen des Wirkstoffes in einer Aufwandmenge, die 1,2, 0,6 und 0,3 kg/ha im Feld entspräche, gespritzt. Unbehandelte Pflanzen wurden als Kontrolle belassen. Die Ausertung des Versuches erfolgte 3, 7 und 14 Tage nach Applikation der Wirksubstanz durch Bestimmen des Grades an Defoliation (% abgefallene Blätter) und an Dessikation (% Austrocknung der an der Pflanze verbleibenden Blätter).

In diesem Versuch beliessen die geprüften Verbindungen bei Aufwandmengen von 0,6 und 1,2 kg/ha nach 7 Tagen bloss noch wenige vertrocknete Blätter auf den Pflanzen (> 80 % Blattfall und Austrocknung).

## Patentansprüche

1. Derivate des N-phenyl-3,4,5,6-tetrahydrophthalsäureimides der Formel I

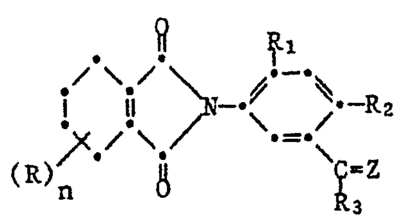

(I),

worin

| | |
|---|---|
| R | $C_1$-$C_3$-Alkyl, |
| n | Null, eins oder zwei, |
| $R_1$ | Fluor |
| $R_2$ | Wasserstoff oder Halogen, |
| Z | Sauerstoff oder einen Oximrest =N-O-Q, der Rest |

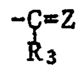

kann auch einen Ketalrest

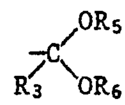

darstellen,

$R_3$ falls Z einen Oximrest darstellt, Methyl oder Cyan,

$R_3$ Wenn Z Sauerstoff oder einen Ketalrest darstellt Wasserstoff, Cyan, $C_1$-$C_4$-Alkyl, $C_1$-$C_4$-Halogenalkyl oder einen Rest $-CH_2YR_4$

Y Sauerstoff oder Schwefel,

$R_4$ $C_1$-$C_4$-Alkyl, $C_3$-$C_4$-Alkenyl, $C_3$-$C_4$-Alkinyl, $C_3$-$C_7$-Cycloalkyl, Benzyl oder Phenyl, wobei der Phenylring unsubstituiert oder durch $C_1$-$C_4$-Alkyl, $C_1$-$C_4$-Alkoxy, $C_1$-$C_4$-Halogenalkyl, $C_1$-$C_4$-Halogenalkoxy, Nitro oder Cyan substituiert sein kann,

$R_5$ und $R_6$ unabhängig voneinander je $C_1$-$C_4$-Alkyl, $C_1$-$C_4$-Halogenalkyl, oder $C_2$-$C_8$-Alkoxyalkyl oder

$R_5$ und $R_6$ zusammen eine Aethylenbrücke, eine Propylenbrücke oder einen über benachbarte Kohlenstoffatome gebundenen Cyclohexylrest bedeuten, wobei die Propylenbrücke und der Cyclohexlyrest bis zu dreimal durch $C_1$-$C_4$-Alkylreste substituiert sein können, während die Aethylenbrücke ein- oder zweimal durch $C_1$-$C_4$-Alkyl, $C_1$-$C_4$-Halogenalkyl, unsubstituiertes oder durch Halogen oder $C_1$-$C_4$-Alkyl Substituiertes Phenyl oder Benzyl, einen Rest-$CH_2YH$ oder-$CH_2Y$ $R_4$ substituiert sein kann,

Q Wasserstoff,

einen $C_1$-$C_{12}$-Alkylrest, der durch Sauerstoff, Schwefel, -CO-, -CONH- oder -NH- unterbrochen oder durch Halogen oder Cyan substituiert sein kann,
einen $C_3$-$C_8$-Alkenyl- oder -Halogenalkenylrest,
einen $C_3$-$C_8$-Alkinylrest,
einen gegebenenfalls durch Halogen substituierten $C_3$-$C_7$-Cycloalkylrest,
einen Phenyl-$C_1$-$C_4$-alkylrest, dessen Phenylkern durch Halogen, $C_1$-$C_4$-Alkyl, $C_1$-$C_4$-Alkoxy, $C_1$-$C_4$-Halogenalkyl, $C_1$-$C_4$-Halogenalkoxy, Nitro oder Cyan substituiert sein kann,
einen $C_1$-$C_4$-Alkoxycarbonylrest,
einen $C_1$-$C_4$-Alkoxythiocarbonylrest,
einen Benzoyl-, Furoyl- oder Thienoylrest, der im Ring unsubstituiert oder durch Halogen, $C_1$-$C_4$-Alkyl, $C_1$-$C_4$-Alkoxy, $C_1$-$C_4$-Halogenalkoxy, Nitro oder Cyan substituiert ist,
einen $C_1$-$C_4$-Alkylsulfonylrest, einen Sulfonamido- oder Carbamoylrest, der unsubstituiert oder durch $C_1$-$C_4$-Alkylrest (e) substituiert ist,
ein Metallion oder ein quaternäres Ammoniumion,
einen über COO- resp. CSO- gebundenen Phenyl- oder Benzylrest bedeutet

2. Derivate des N-Phenyl-3,4,5,6-tetrahydrophthalsäureimides gemäss Anspruch 1 der Formel Ia

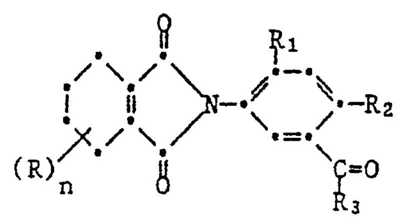

(Ia),

worin
$R_3$, $C_1$-$C_4$-Alkyl oder einen Rest-$CH_2YR_4$ bedeutet während n, R, $R_1$, $R_2$ und $R_4$ die im Anspruch 1 gegebene Bedeutung haben sowie die Stereoisomeren und optisch aktiven Enantiomere dieser Derivate.

3. 2-Chlor-4-fluor-5-(N-3,4,5,6-tetrahydrophthalsäureimido)-acetophenongemäss Anspruch 1.

**4.** 4-Fluor-5-(N-3,4,5,6-tetrahydrophthalsäureimido)-acetophenon gemäss Anspruch 1.

**5.** 2-Brom-4-fluor-5-(N-2,3,4,5-tetrahydrophthalsäureimido)-acetophenon gemäss Anspruch 1.

**6.** Derivate des N-Phenyl-3,4,5,6-tetrahydrophthalsäureimides gemäss Anspruch 1 der Formel Ib

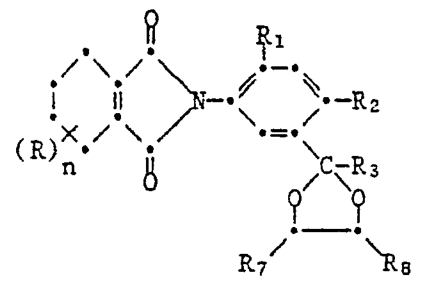

(Ib),

worin $R_3$, $C_1$-$C_4$ alkyl $R_7$ und $R_8$ unabhängig voneinander je Wasserstoff oder $C_1$-$C_4$-Alkyl bedeuten und n, R, $R_1$ und $R_2$ die unter der Formel I gegebene Bedeutung haben.

**7.** N-[2-Fluor-4-chlor-5-(2-methyl-1,3-dioxolan-2-yl)-phenyl]-3,4,5,6-tetrahydrophthalsäureimid gemäss Anspruch 1.

**8.** N-[2-Fluor-4-chlor-5-(2,4-dimethyl-1,3-dioxolan-2-yl)-phenyl]-3,4,5,6-tetrahydrophthalsäureimid gemäss Anspruch 1.

**9.** N-[2-Fluor-4-chlor-5-(4-äthyl-2-methyl-1,3-dioxolan-2-yl)-phenyl]-3,4,5,6-tetrahydrophthalsäureimid gemäss Anspruch 1.

**10.** N-[2,4-Difluor-5-(2,4-dimethyl-1,3-dioxolan-2-yl)-phenyl]-3,4,5,6-tetrahydrophthalsäureimid gemäss Anspruch 1.

**11.** N-[2,4-Difluor-5-(2,4,5-trimethyl-1,3-dioxolan-2-yl)-phenyl]-3,4,5,6-tetrahydrophthalsäureimid gemäss Anspruch 1.

**12.** N-[2-Fluor-4-chlor-5-(2,4,5-trimethyl)-1-3-dioxolan-2-yl)-phenyl]-3,4,5,6-tetrahydrophthalsäureimid gemäss Anspruch 1.

**13.** Derivate des N-Phenyl-3,4,5,6-tetrahydrophthalsäureimides gemäss Anspruch 1 der Formel Ic

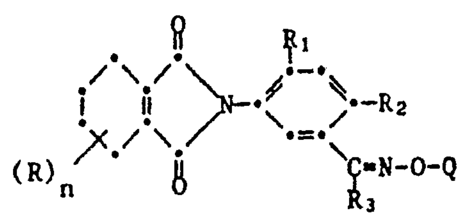

(Ic)

worin
n, $R_1$ und $R_2$ die im Anspruch 1 gegebene Bedeutung haben, $R_3$ Methyl oder Cyan und Q Wasserstoff, einen $C_1$-$C_{10}$-Alkylrest, der durch Sauerstoff, Schwefel, oder -NH- unterbrochen sein kann, oder der durch Halogen Cyan, substituiert sein kann; einen $C_3$-$C_8$-Alkenyl oder Halogenalkenylrest, einen $C_3$-$C_8$-Alkinylrest, einen Phenyl-$C_1$-$C_4$-alkylrest, dessen Phenylkern durch Halogen, $C_1$-$C_4$-Alkyl, $C_1$-$C_4$-Alkoxy, $C_1$-$C_4$-Halogenalkoxy, Nitro oder Cyan substituiert sein kann; einen $C_1$-$C_4$-Alkylcarbonylrest, einen $C_1$-$C_4$-Alkylthiocarbonylrest, einen $C_1$-$C_4$-Alkylsulfonylrest oder einen $C_1$-$C_4$-Alkylcarbamoyl-bedeuten sowie die Stereoisomeren und optisch aktiven Enantiomere dieser Derivate.

**14.** 2-Chlor-4-fluor-5-(N-3,4,5,6-tetrahydrophthalsäureimido)-acetophenonoxim gemäss Anspruch 1.

15. 4-Fluor-5-(N-3,4,5,6-tetrahydrophtalsäureimido)-acetophenonoxim gemäss Anspruch 1.

16. 2-Chlor-4-fluor-5-(N-3,4,5,6-tetrahydrophthalsäureimido)-acetophenonoxim-(methoxy-carbonyl-äth-1-yl)-äther gemäss Anspruch 1.

17. 4-Fluor-5-(N-3,4,5,6-tetrahydrophthalsäureimido)-acetophenonoxim-(methoxy-carbonyl-äth-1-yl)-äther gemäss Anspruch 1.

18. 2-Chlor-4-fluor-5-(N-3,4,5,6-tetrahydrophthalsäureimido)-acetophenonoxim-methyl-äther gemäss Anspruch 1.

19. 2-Chlor-4-fluor-5-(N-3,4,5,6-tetrahydrophthalsäureimido)-acetophenonoxim-äthyl-äther gemäss Anspruch 1.

20. 2-Chlor-4-fluor-5-(N-3,4,5,6-tetrahydrophthalsäureimido)-acetophenonoxim-n-butyl-äther gemäss Anspruch 1.

21. 2-Chlor-4-fluor-5-(N-3,4,5,6-tetrahydrophthalsäureimido)-acetophenonoxim-t-butyl-äther gemäss Anspruch 1.

22. 2-Chlor-4-fluor-5-(N-3,4,5,6-tetrahydrophthalsäureimido)-acetophenonoxim-benzyl-äther gemäss Anspruch 1.

23. 2-Chlor-4-fluor-5-(N-3,4,5,6-tetrahydrophthalsäureimido)-acetophenonoxim-(2-chlorbenzyl)-äther gemäss Anspruch 1.

24. 2,4-Difluor-5-(N-3,4,5,6-tetrahydrophthalsäureimido)-acetophenonoxim-(2-chlorbenzyl)-äther gemäss Anspruch 1.

25. Derivate des N-Phenyl-3,4,5,6-tetrahydrophthalsäureimides gemäss Anspruch 1 der Formel Ic

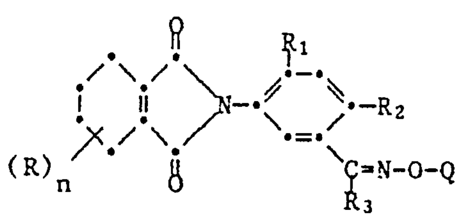

(Ic)

worin n Null, $R_3$ Cyan, Q $C_1$-$C_{10}$-Alkyl unsubstituiert oder durch Halogen oder $C_1$-$C_6$-Alkoxycarbonyl substituiert; $C_3$-$C_8$-Alkenyl oder Halogenalkenyl; Benzoyl oder Halogenbenzoyl bedeuten, während $R_1$ und $R_2$ die im Anspruch 1 gegebene Bedeutung haben.

26. Methoximino-[2-chlor-4-fluor-5-(N-3,4,5,6-tetrahydrophthalsäureimido)]-benzoyl-cyanid gemäss Anspruch 1.

27. Aethoximino-[2-chlor-4-fluor-5-(N-3,4,5,6-tetrahydrophthalsäureimido)]-benzoyl-cyanid gemäss Anspruch 1.

28. Isopropoximino-[2-chlor-4-fluor-5-(N-3,4,5,6-tetrahydrophthalsäureimido)]-benzoyl-cyanid gemäss Anspruch 1.

29. sec.-Butoximino-[2-chlor-4-fluor-5-(N-3,4,5,6-tetrahydrophthalsäureimido)]-benzoyl-cyanid gemäss Anspruch 1.

30. Methoxycarbonyl-äth-1-oximino-[2-chlor-4-fluor-5-(N-3,4,5,6-tetrahydrophthalsäureimido)]-benzoyl-cyanid gemäss Anspruch 1.

**31.** Isopropoxycarbonyl-äth-1-oximino-[2-chlor-4-fluor-5-(N-3,4,5,6-tetrahydrophthalsäureimido)]-benzoylcyanid gemäss Anspruch 1.

**32.** Verfahren zur Herstellung der 5-(N-3,4,5,6-Tetrahydrophthalsäureimido)-phenonderivate der Formel I, gemäss Anspruch 1, dadurch gekennzeichnet, dass man das Anhydrid einer 3,4,5,6-Tetrahydrophthalsäure der Formel II

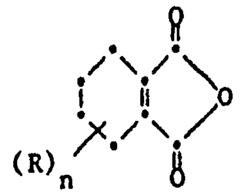

(II)

worin R und n die im Anspruch 1 gegebene Bedeutung haben, in einem inerten organischen Lösungs- oder Verdünnungsmittel, gegebenenfalls in Gegenwart einer geringen Menge eines wasserentziehenden Mittels, mit der äquimolaren Menge eines 3-Aminophenon-derivates der Formel V

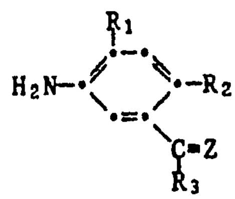

(V)

worin $R_1$, $R_2$, $R_3$ und Z die im Anspruch 1 gegebene Bedeutung haben, umsetzt.

**33.** Verfahren zur Herstellung der Derivate des 3,4,5,6-Tetrahydrophthalsäureimides der Formel Ia, gemäss Anspruch 2, dadurch gekennzeichnet, dass man das Anhydrid einer 3,4,5,6-Tetrahydrophthalsäure der Formel II

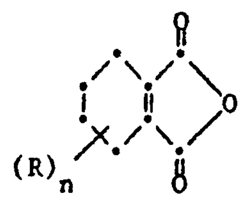

(II)

worin R und n die unter der Formel I gegebene Bedeutung haben, in einem inerten organischen Lösungs- oder Verdünnungsmittel, gegebenenfalls in Gegenwart einer geringen Menge eines wasserentziehenden Mittels, mit der äquimolaren Menge einer 3-Aminobenzoylverbindung der Formel III

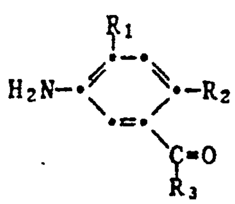

(III)

worin $R_1$, $R_2$ und $R_3$ die im Anspruch 1 gegebene Bedeutung haben, umsetzt und gegebenenfalls, wenn Z eine von Sauerstoff verschiedene Bedeutung hat, dieses Produkt noch in einem inerten Lösungsmittel, in Gegenwart einer Säure mit Alkohol oder einem Diol der Formeln IV resp. IVa

$R_5$-OH (IV) resp. HO-$R_5$-$R_6$-OH (IVa)

worin $R_5$ und $R_6$ die im Anspruch 1 gegebene Bedeutung haben, umsetzt.

**34.** Verfahren zur Herstellung der Derivate des N-Phenyl-3,4,5,6-tetrahydrophthalsäureimides der Formel Ic gemäss Anspruch 15, dadurch gekennzeichnet, dass man ein Salz der Formel VII

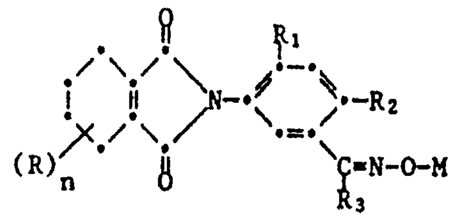

(VII),

in welcher M ein Alkalimetall- oder Erdalkalimetallkation darstellt und n, R, $R_1$, $R_2$ und $R_3$ die oben angegebene Bedeutung haben, mit einem reaktionsfähigen Ester der Formel VIII umsetzt

Y - Q (VIII)

worin Q die unter Formel I gegebene Bedeutung hat und Y einen organischen oder anorganischen Säurerest darstellt.

**35.** Ein herbizides und Pflanzenwachstum regulierendes Mittel, dadurch gekennzeichnet, dass es neben Träger- und/oder anderen Zuschlagstoffen als Wirkstoff ein Oximderivat des N-Phenyl-3,4,5,6-tetrahydrophtalsäureimides der Formel I, Anspruch 1 enthält.

**36.** Die Verwendung eines Wirkstoffes gemäss Anspruch 1, oder einen solchen Wirkstoff enthaltendes Mittel zur Bekämpfung unerwünschten Pflanzenwachstums.

**37.** Die Verwendung eines Wirkstoffes gemäss Anspruch 1 oder einen solchen Wirkstoff enthaltendes Mittel zur Hemmung des Pflanzenwachstums.

**38.** Die Verwendung eines Wirkstoffes gemäss Anspruch 1, oder einen solchen Wirkstoff enthaltendes Mittel zur Beeinflussung des Pflanzenwachstums zum Zweck der Ertragssteigerung.

**39.** Verfahren zur selektiven pre- oder postemergenten Bekämpfung von Unkräutern in Nutzpflanzenkulturen, dadurch gekennzeichnet, dass man die Nutzpflandzenkulturen oder deren Anbaufläche mit einer wirksamen Menge eines Wirkstoffes der Formel I, Anspruch 1 oder eines eine solche Verbindung enthaltenden Mittels behandelt.

**40.** Verfahren zur Unterdrückung des Pflanzenwachstums über das 2-Blattstadium hinaus, dadurch gekennzeichnet, dass man die Pflanzen während ihres Wachstums mit einer wirksamen Menge eines Wirkstoffes der Formel I Anspruch 1 oder eines einen solchen Wirkstoff enthaltenden Mittels behandelt.

**41.** 3-Aminophenoxime der Formel Va als Zwischenprodukte

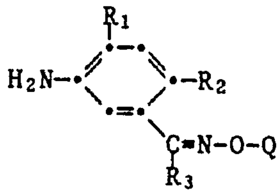

Va

worin

$R_1$ Fluor

$R_2$ Chlor oder Brom

$R_3$ Cyan
Q Wasserstoff, ein Alkalimetallion, $C_1$-$C_{10}$-Alkyl unsubstituiert oder durch Halogen, Cyan, $C_1$-$C_6$-Alkoxy oder $C_1$-$C_6$-Alkoxycarbonyl substituiert; $C_3$-$C_8$-Cycloalkyl; $C_3$-$C_8$-Alkenyl oder $C_3$-$C_8$-Halogenoalkenyl; Benzoyl oder Halogenobenzoyl bedeuten.

**42.** Methoximino-5-amino-2-chlor-4-fluorbenzoylcyanid gemäss Anspruch 45.

**43.** Natrium-oximino-5-amino-2-chlor-4-fluorbenzoylcyanid gemäss Anspruch 45.

**44.** Verfahren zur Herstellung von 3-Aminophenonoximen der Formel Va gemäss Anspruch 45, dadurch gekennzeichnet, dass man ein durch $R_1$ und $R_2$ substituiertes Benzylcyanid der Formel Vb

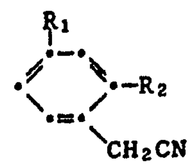

Vb

worin $R_1$ und $R_2$ die im Anspruch 45 gegebene Bedeutung haben, in einem inerten organischen Lösungs- oder Verdünnungsmittel in Gegenwart von Natriumäthylat mit Isopentylnitrit behandelt, und das entstandene Cyanoacetoxim-Natrium Salz der Formel Vc

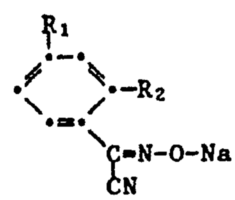

Vc

worin $R_1$ und $R_2$ die im Anspruch 45 gegebene Bedeutung haben, gegebenenfalls in situ mit einem Halogenid der Formel

QHal

wobei Hal ein Halogenatom bedeutet und Q die im Anspruch 45 gegebene Bedeutung hat, veräthert und den erhaltenen Phenoximäther der Formel Vd

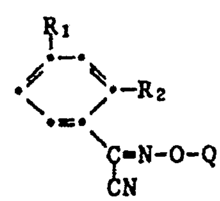

Vd

worin $R_1$, $R_2$ und Q die im Anspruch 45 gegebene Bedeutung haben, mit Salpetersäure behandelt und den erhaltenen 3-Nitrophenoximäther der Formel Ve

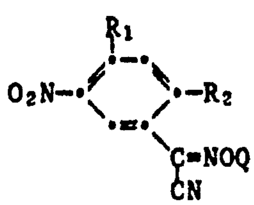

Ve

worin $R_1$, $R_2$ und Q die im Anspruch 45 gegebene Bedeutung haben, in wässriger Salzsäure mit Zinnchlorid zum 3-Aminophenoximäther der Formel Va gemäss Anspruch 45 reduziert.

## Claims

1. A derivative of the N-phenyl-3,4,5,6-tetrahydrophthalimide of the formula I

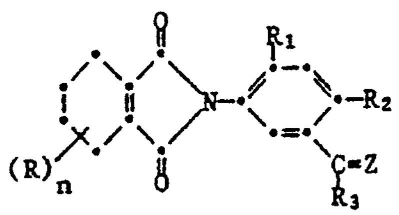

(I)

in which

| | |
|---|---|
| R | is $C_1$-$C_3$alkyl, |
| n | is zero, one or two, |
| $R_1$ | is fluorine, |
| $R_2$ | is hydrogen or halogen, |
| Z | is oxygen or an oxime radical =N-O-Q, and the radical |

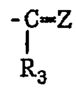

can also be a ketal radical

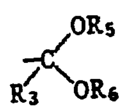

,

| | |
|---|---|
| $R_3$ | is methyl or cyano, if Z is an oxime radical, |
| $R_3$ | is hydrogen, cyano, $C_1$-$C_4$alkyl, $C_1$-$C_4$haloalkyl or a radical $-CH_2YR_4$, if Z is oxygen or a ketal radical, |
| Y | is oxygen or sulfur, |
| $R_4$ | is $C_1$-$C_4$alkyl, $C_3$-$C_4$alkenyl, $C_3$-$C_4$alkynyl, $C_3$-$C_7$cycloalkyl, benzyl or phenyl, and the phenyl ring can be unsubstituted or substituted by $C_1$-$C_4$alkyl, $C_1$-$C_4$alkoxy, $C_1$-$C_4$haloalkyl, $C_1$-$C_4$haloalkoxy, nitro or cyano, |
| $R_5$ and $R_6$ | independently of one another are each $C_1$-$C_4$alkyl, $C_1$-$C_4$haloalkyl or $C_2$-$C_8$alkoxyalkyl, or |
| $R_5$ and $R_6$ | together are an ethylene bridge, a propylene bridge, or a cyclohexyl radical bound by way of adjacent carbon atoms, and the propylene bridge and the cyclohexyl radical can be substituted up to three times by $C_1$-$C_4$alkyl radicals, while the ethylene bridge can be mono- or disubstituted by $C_1$-$C_4$alkyl, $C_1$-$C_4$haloalkyl, or by benzyl or phenyl unsubstituted or substituted by halogen or $C_1$-$C_4$alkyl, or can be mono- or disubstituted by a radical $-CH_2YH$ or $-CH_2YR_4$, and |
| Q | is hydrogen, a $C_1$-$C_{12}$alkyl radical which can be interrupted by oxygen, sulfur, -CO-, -CONH- or -NH-, or which can be substituted by halogen or cyano, or is a $C_3$-$C_8$alkenyl or $C_3$-$C_8$haloalkenyl radical, a $C_3$-$C_8$alkynyl radical, a $C_3$-$C_7$cycloalkyl radical unsubstituted or substituted by halogen, or is a phenyl-$C_1$-$C_4$alkyl radical, the phenyl nucleus of which can be substituted by halogen, $C_1$-$C_4$alkyl, $C_1$-$C_4$alkoxy, $C_1$-$C_4$haloalkyl, $C_1$-$C_4$haloalkoxy, nitro or cyano, or is a $C_1$-$C_4$alkoxycarbonyl radical, or is |

a $C_1$-$C_4$-alkoxythiocarbonyl radical, or is a benzoyl, furoyl or thienoyl radical, which in the ring is unsub-stituted or substituted by halogen, $C_1$-$C_4$alkyl, $C_1$-$C_4$alkoxy, $C_1$-$C_4$haloalkoxy, nitro or cyano, or is a $C_1$-$C_4$alkylsulfonyl radical, a sulfonamido or carbamoyl radical, which is unsubstituted or substituted by $C_1$-$C_4$alkyl radical(s), or is a metal ion or a quaternary ammonium ion, or is a phenyl or benzyl radical bound via COO- or CSO-.

2. A derivative of the N-phenyl-3,4,5,6-tetrahydrophthalimide according to claim 1 of the formula Ia

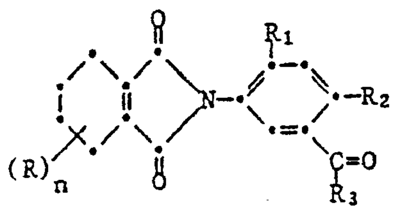

(Ia)

in which
$R_3$ is $C_1$-$C_4$alkyl or a radical $-CH_2YR_4$, and n, R, $R_1$, $R_2$ and $R_4$ have the meanings defined in claim 1, and the stereoisomers and optically active enantiomers of this derivative.

3. 2-Chloro-4-fluoro-5-(N-3,4,5,6-tetrahydrophthalimido)-acetophenone according to claim 1.

4. 4-Fluoro-5-(N-3,4,5,6-tetrahydrophthalimido)-acetophenone according to claim 1.

5. 2-Bromo-4-fluoro-5-(N-2,3,4,5-tetrahydrophthalimido)-acetophenone according to claim 1.

6. A derivative of N-phenyl-3,4,5,6-tetrahydrophthalimide according to claim 1 of the formula Ib

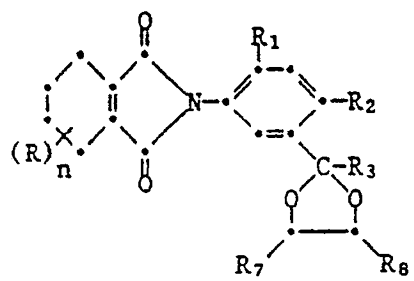

(Ib)

in which
$R_3$ is $C_1$-$C_4$alkyl and $R_7$ and $R_8$ independently of one another are each hydrogen or $C_1$-$C_4$alkyl, and n, R, $R_1$ and $R_2$ have the meanings defined under the formula I.

7. N-[2-Fluoro-4-chloro-5-(2-methyl-1,3-dioxolan-2-yl)-phenyl]-3,4,5,6-tetrahydrophthalimide according to claim 1.

8. N-[2-Fluoro-4-chloro-5-(2,4-dimethyl-1,3-dioxolan-2-yl)-phenyl]-3,4,5,6-tetrahydrophthalimide according to claim 1.

9. N-[2-Fluoro-4-chloro-5-(4-ethyl-2-methyl-1,3-dioxolan-2-yl)-phenyl]-3,4,5,6-tetrahydrophthalimide according to claim 1.

10. N-[2,4-Difluoro-5-(2,4-dimethyl-1,3-dioxolan-2-yl)-phenyl]-3,4,5,6-tetrahydrophthalimide according to claim 1.

11. N-[2,4-Difluoro-5-(2,4,5-trimethyl-1,3-dioxolan-2-yl)phenyl]-3,4,5,6-tetrahydrophthalimide according to claim 1.

12. N-[2-Fluoro-4-chloro-5-(2,4,5-trimethyl)-1,3-dioxolan-2-yl)phenyl]-3,4,5,6-tetrahydrophthalimide according to claim 1.

13. A derivative of the N-phenyl-3,4,5,6-tetrahydrophthalimide according to claim 1 of the formula Ic

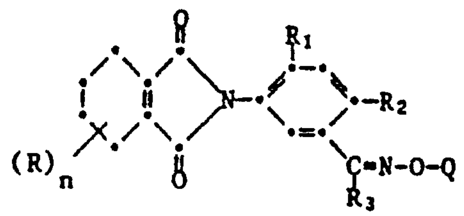

(Ic)

in which

| | |
|---|---|
| n, $R_1$ and $R_2$ | have the meanings defined in claim 1, |
| $R_3$ | is methyl or cyano, and |
| Q | is hydrogen, a $C_1$-$C_{10}$alkyl group which can be interrupted by oxygen, sulfur or -NH-, or can be substituted by halogen or cyano, or is a $C_3$-$C_8$alkenyl or $C_3$-$C_8$haloalkenyl radical, a $C_3$-$C_8$alkynyl radical, a phenyl-$C_1$-$C_4$alkyl radical of which the phenyl nucleus can be substituted by halogen, $C_1$-$C_4$alkyl, $C_1$-$C_4$alkoxy, $C_1$-$C_4$haloalkoxy, nitro or cyano, or is a $C_1$-$C_4$alkylcarbonyl radical, a $C_1$-$C_4$alkylthiocarbonyl radical, a $C_1$-$C_4$alkylsulfonyl radical or a $C_1$-$C_4$alkylcarbamoyl radical, |

and the stereoisomers and optically active enantiomers of this derivative.

14. 2-Chloro-4-fluoro-5-(N-3,4,5,6-tetrahydrophthalimido)-acetophenone oxime according to claim 1.

15. 4-Fluoro-5-(M-3,4,5,6-tetrahydrophthalimido)-acetophenone oxime according to claim 1.

16. 2-Chloro-4-fluoro-5-(N-3,4,5,6-tetrahydrophthalimido)-acetophenone oxime methoxycarbonyleth-1-yl ether according to claim 1.

17. 4-Fluoro-5-(N-3,4,5,6-tetrahydrophthalimido)-acetophenone oxime methoxycarbonyleth-1-yl ether according to claim 1.

18. 2-Chloro-4-fluoro-5-(N-3,4,5,6-tetrahydrophthalimido)-acetophenone oxime methyl ether according to claim 1.

19. 2-Chloro-4-fluoro-5-(N-3,4,5,6-tetrahydrophthalimido)-acetophenone oxime ethyl ether according to claim 1.

20. 2-Chloro-4-fluoro-5-(N-3,4,5,6-tetrahydrophthalimido)-acetophenone oxime n-butyl ether according to claim 1.

21. 2-Chloro-4-fluoro-5-(N-3,4,5,6-tetrahydrophthalimido)-acetophenone oxime t-butyl ether according to claim 1.

22. 2-Chloro-4-fluoro-5-(N-3,4,5,6-tetrahydrophthalimido)-acetophenone oxime benzyl ether according to claim 1.

23. 2-Chloro-4-fluoro-5-(N-3,4,5,6-tetrahydrophthalimido)-acetophenone oxime 2-chlorobenzyl ether according to claim 1.

24. 2,4-Difluoro-5-(N-3,4,5,6-tetrahydrophthalimido)-acetophenone oxime 2-chlorobenzyl ether according to claim 1.

25. A derivative of the N-phenyl-3,4,5,6-tetrahydrophthalimide according to claim 1 of the formula Ic

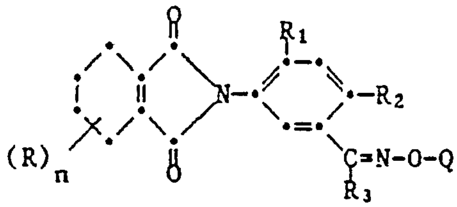

(Ic)

in which n is zero, $R_3$ is cyano, and Q is $C_1$-$C_{10}$alkyl, which is unsubstituted or substituted by halogen or $C_1$-$C_6$alkoxycarbonyl, or is $C_3$-$C_8$alkenyl or $C_3$-$C_8$haloalkenyl, benzoyl or halobenzoyl, while $R_1$ and $R_2$ have the meanings defined in claim 1.

**26.** Methoximino-[2-chloro-4-fluoro-5-(N-3,4,5,6-tetrahydrophthalimido)]-benzoyl cyanide according to claim 1.

**27.** Ethoximino-[2-chloro-4-fluoro-5-(N-3,4,5,6-tetrahydrophthalimido)]-benzoyl cyanide according to claim 1.

**28.** Isopropoximino-[2-chloro-4-fluoro-5-(N-3,4,5,6-tetrahydrophthalimido)]-benzoyl cyanide according to claim 1.

**29.** sec-Butoximino-[2-chloro-4-fluoro-5-(N-3,4,5,6-tetrahydrophthalimido)]-benzoyl cyanide according to claim 1.

**30.** Methoxycarbonyl-eth-1-oximino-(2-chloro-4-fluoro-5-(N-3,4,5,6-tetrahydrophthalimido)]-benzoyl cyanide according to claim 1.

**31.** Isopropoxycarbonyl-eth-1-oximino-[2-chloro-4-fluoro-5-(N-3,4,5,6-tetrahydrophthalimido)]-benzoyl cyanide according to claim 1.

**32.** A process for preparing a 5-(N-3,4,5,6-tetrahydrophthalimido)-phenone derivative of the formula I according to claim 1, which comprises reacting the anhydride of a 3,4,5,6-tetrahydrophthalic acid of the formula II

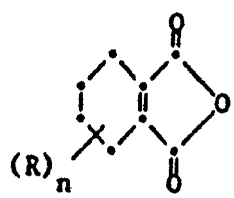

(II),

in which R and n have the meanings defined in claim 1, in an inert organic solvent or diluent, optionally in the presence of a small amount of a dehydrating agent, with the equimolar amount of a 3-aminophenone derivative of the formula V

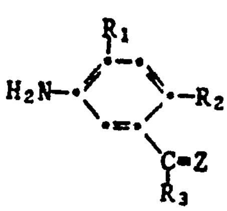

(V)

in which $R_1$, $R_2$, $R_3$ and Z have the meanings defined in claim 1.

**33.** A process for preparing a derivative of the 3,4,5,6-tetrahydrophthalimide of the formula Ia according to

claim 2, which comprises reacting the anhydride of a 3,4,5,6-tetrahydrophthalic acid of the formula II

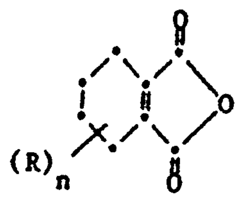

(II),

in which R and n have the meanings defined under the formula I, in an inert organic solvent or diluent, optionally in the presence of a small amount of a dehydrating agent, with the equimolar amount of a 3-aminobenzoyl compound of the formula III

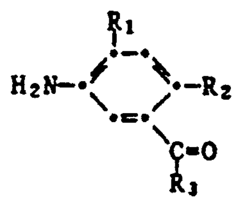

(III),

in which $R_1$, $R_2$ and $R_3$ have the meanings defined in claim 1; and optionally, where Z has a meaning other than oxygen, reacting this product, in an inert solvent in the presence of an acid, with an alcohol or a diol of the formulae IV and IVa respectively

$R_5$-OH (IV) and HO-$R_5$-$R_6$-OH (IVa),

in which $R_5$ and $R_6$ have the meanings defined in claim 1.

**34.** A process for preparing a derivative of the N-phenyl-3,4,5,6-tetrahydrophthalimide of the formula Ic according to claim 15, which comprises reacting a salt of the formula VII

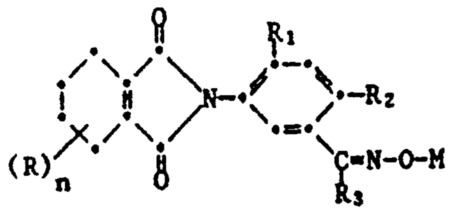

(VII),

in which M is an alkali metal cation or alkaline-earth metal cation, and n, R, $R_1$, $R_2$ and $R_3$ have the meanings defined above, with a reactive ester of the formula VIII

Y - Q (VIII)

in which Q has the meaning defined under formula I and Y is an organic or inorganic acid radical.

**35.** A herbicidal and plant-growth-regulating composition which contains as active substance an oxime derivative of the N-phenyl-3,4,5,6-tetrahydrophthalimide of the formula I, claim 1, in addition to carriers and/or other additives.

**36.** The use of an active substance according to claim 1, or composition containing such an active substance, for controlling unwanted plant growth.

**37.** The use of an active substance according to claim 1, or composition containing such an active substance, for inhibiting plant growth.

**38.** The use of an active substance according to claim 1, or composition containing such an active substance, for influencing plant growth for the purpose of increasing the yield.

**39.** A method of selectively controlling, before or after emergence, weeds in crops of cultivated plants, which comprises treating the crops or the cultivated area thereof with an effective amount of an active substance of the formula I, claim 1, or of a composition containing such a compound.

**40.** A method of suppressing plant growth beyond the 2-leaf stage, which comprises treating the plants during their growth with an effective amount of an active substance of the formula I, claim 1, or of a composition containing such an active substance.

**41.** A 3-aminophenoxime of the formula Va as intermediate

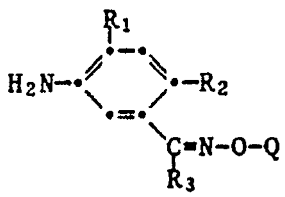

(Va)

in which
- $R_1$ is fluorine,
- $R_2$ is chlorine or bromine,
- $R_3$ is cyano,
- Q is hydrogen, an alkali metal ion, $C_1$-$C_{10}$alkyl unsubstituted or substituted by halogen, cyano, $C_1$-$C_6$alkoxy or $C_1$-$C_6$alkoxycarbonyl, or is $C_3$-$C_8$cycloalkyl, $C_3$-$C_8$alkenyl or $C_3$-$C_8$haloalkenyl, benzoyl or halobenzoyl.

**42.** Methoximino-5-amino-2-chloro-4-fluorobenzoyl cyanide according to claim 41.

**43.** Sodium oximino-5-amino-2-chloro-4-fluorobenzoyl cyanide according to claim 41.

**44.** A process for preparing a 3-aminophenone oxime of the formula Va according to claim 41, which comprises treating a benzyl cyanide of the formula Vb, substituted by $R_1$ and $R_2$,

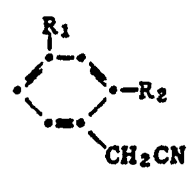

(Vb),

in which $R_1$ and $R_2$ have the meanings defined in claim 41, in an inert organic solvent or diluent in the presence of sodium ethylate, with isopentyl nitrite, and etherifying the resultant cyanoacetoxime sodium salt of the formula Vc

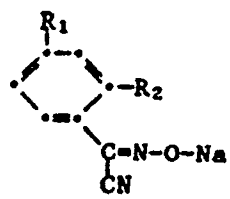

(Vc),

in which $R_1$ and $R_2$ have the meanings defined in claim 41, optionally in situ, with a halide of the formula

Q-Hal ,

in which Hal is a halogen atom, and Q has the meaning given in claim 41, and treating the resulting phenoxime ether of the formula Vd

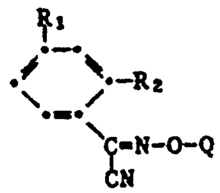

(Vd) ,

in which $R_1$, $R_2$ and Q have the meanings defined in claim 41, with nitric acid, and reducing the resulting 3-nitrophenoxime ether of the formula Ve

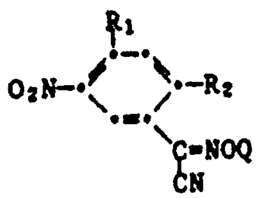

(Ve) ,

in which $R_1$, $R_2$ and Q have the meanings defined in claim 41, in aqueous hydrochloric acid, with tin chloride to obtain the 3-aminophenoxime ether of the formula Va according to claim 41.

**Revendications**

**1.** Dérivés du N-phényl-3,4,5,6-tétrahydrophtalimide de formule I

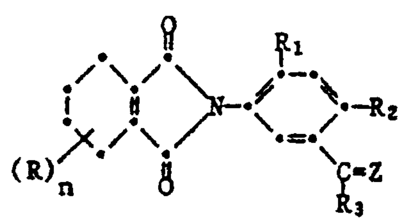

(I) ,

dans laquelle

| | |
|---|---|
| R | représente un groupe alkyle en C 1-C 3, |
| n | est égal à 0, 1 ou 2, |
| $R_1$ | représente le fluor, |
| $R_2$ | représente l'hydrogène ou un halogène, |
| Z | représente l'oxygène ou un radical d'oxime =N-O-Q, mais le groupe |

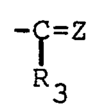

peut également représenter un radical d'acétal

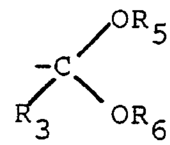 ,

| | |
|---|---|
| $R_3$ | représente un groupe méthyle ou cyano lorsque Z représente un radical d'oxime, |
| $R_3$ | représente l'hydrogène, un groupe cyano, alkyle en C 1-C 4, halogénoalkyle en C 1-C 4 ou un groupe $-CH_2YR_4$ lorsque Z représente l'oxygène ou un radical d'acétal, |
| Y | représente l'oxygène ou le soufre, |
| $R_4$ | représente un groupe alkyle en C 1-C 4, alcényle en C 3-C 4, alcynyle en C 3-C 4, cycloalkyle en C 3-C 7, benzyle ou phényle dans lesquels le noyau phényle peut être non substitué ou substitué par des groupes alkyle en C 1-C 4, alcoxy en C 1-C 4, halogénoalkyle en C 1-C 4, halogénoalcoxy en C 1-C 4, nitro ou cyano, |
| $R_5$ et $R_6$ | représentent chacun, indépendamment l'un de l'autre, un groupe alkyle en C 1-C 4, halogénoalkyle en C 1-C 4 ou alcoxyalkyle en C 2-C 8, ou bien |
| $R_5$ et $R_6$ | forment ensemble un pont éthylène, un pont propylène ou un groupe cyclohexyle relié par des atomes de carbone voisins, le pont propylène et le groupe cyclohexyle pouvant porter jusqu'à trois substituants alkyle en C 1-C 4, et le pont éthylène pouvant être substitué une ou deux fois par des groupes alkyle en C 1-C 4, halogénoalkyle en C 1-C 4, phényle ou benzyle eux-mêmes non substitués ou substitués par des halogènes ou des groupes alkyle en C 1-C 4, un groupe $-CH_2YH$ ou $-CH_2YR_4$, |
| Q | représente l'hydrogène, |

un groupe alkyle en C 1-C 12 qui peut être interrompu par l'oxygène, le soufre, un groupe -CO-, -CONH- ou -NH-ou substitué par des halogènes ou des groupes cyano,
un groupe alcényle en C 3-C 8 ou halogénoalcényle en C 3-C 8,
un groupe alcynyle en C 3-C 8,
un groupe cycloalkyle en C 3-C 7 éventuellement substitué par des halogènes,
un groupe phényl-alkyle en C 1-C 4 dont le noyau phényl peut être substitué par des halogènes, des groupes alkyle en C 1-C 4, alcoxy en C 1-C 4, halogénoalkyle en C 1-C 4, halogénoalcoxy en C 1-C 4, nitro ou cyano,
un groupe (alcoxy en C 1-C 4)-carbonyle,
un groupe (alcoxy en C 1-C 4)-thiocarbonyle,
un groupe benzoyle, furoyle, thiénoyle qui peut être non substitué ou substitué dans le noyau par des halogènes, des groupes alkyle en C 1-C 4, alcoxy en C 1-C 4, halogénoalcoxy en C 1-C 4, nitro ou cyano,
un groupe alkylsulfonyle en C 1-C 4, sulfonamido ou carbamoyle, non substitué ou substitué par des groupes alkyle en C 1-C 4,
un ion métallique ou un ion d'ammonium quaternaire,
un groupe phényle ou benzyle relié par l'intermédiaire de COO- ou CSO-.

2. Dérivés du N-phényl-3,4,5,6-tétrahydrophtalimide selon revendication 1, de formule Ia

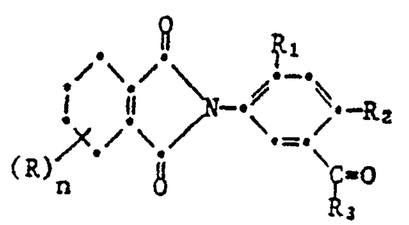

(Ia),

dans laquelle
$R_3$ représente un groupe alkyle en C 1-C 4 ou un groupe $-CH_2YR_4$, et n, R, $R_1$, $R_2$ et $R_4$ ont les significations indiquées dans la revendication 1, ainsi que les stéréoisomères et énantiomères optiques de ces dérivés.

3. La 2-chloro-4-fluoro-5-(N-3,4,5,6-tétrahydrophtalimido)-acétophénone selon revendication 1.

4. La 4-fluoro-5-(N-3,4,5,6-tétrahydrophtalimido)-acétophénone selon revendication 1.

5. La 2-bromo-4-fluoro-5(N-3,4,5,6-tétrahydrophtalimido)-acétophénoneselon revendication 1.

6. Dérivés du N-phényl-3,4,5,6-tétrahydrophtalimide selon revendication 1, de formule Ib

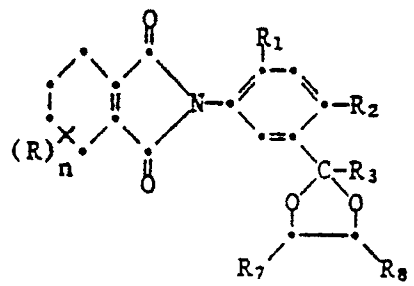

(Ib),

dans laquelle $R_3$ représente un groupe alkyle en C 1-C 4, $R_4$ et $R_8$ représentent chacun, indépendamment l'un de l'autre, l'hydrogène ou un groupe alkyle en C 1-C 4 et n, R, $R_1$ et $R_2$ ont les significations indiquées en référence à la formule I.

7. Le N-[2-fluoro-4-chloro-5-(2-méthyl-1,3-dioxolanne-2-yl)-phényl]-3,4,5,6-tétrahydrophtalimide selon revendication 1.

8. Le N-[2-fluoro-4-chloro-5-(2,4-diméthyl-1,3-dioxolanne-2-yl)-phényl]-3,4,5,6-tétrahydrophtalimide selon revendication 1.

9. Le N-[2-fluoro-4-chloro-5-(4-éthyl-2-méthyl-1,3-dioxolanne-2-yl)-phényl]-3,4,5,6-tétrahydrophtalimide selon revendication 1.

10. Le N-[2,4-difluoro-5-(2,4-diméthyl-1,3-dioxolanne-2-yl)-phényl]-3,4,5,6-tétrahydrophtalimide selon revendication 1.

11. Le N-[2,4-difluoro-5-(2,4,5-triméthyl-1,3-dioxolanne-2-yl)-phényl]-3,4,5,6-tétrahydrophtalimide selon revendication 1.

12. Le N-[2-fluoro-4-chloro-5-(2,4,5-triméthyl-1,3-dioxolanne-2-yl)-phényl]-3,4,5,6-tétrahydrophtalimide selon revendication 1.

13. Dérivés du N-phényl-3,4,5,6-tétrahydrophtalimide selon revendication 1, de formule Ic

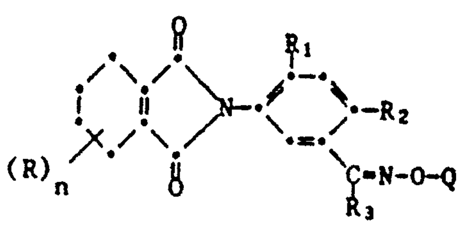

(Ic),

dans laquelle
n, $R_1$ et $R_2$ ont les significations indiquées dans la revendication 1, $R_3$ représente un groupe méthyle ou cyano et Q représente l'hydrogène, un groupe alkyle en C 1-C 10 qui peut être interrompu par l'oxygène, le soufre ou un groupe -NH- ou qui peut être substitué par des halogènes ou des groupes cyano ; un groupe alcényle en C 3-C 8 ou halogénoalcényle, un groupe alcynyle en C 3-C 8, un groupe phényl-alkyle en C 1-C 4 dont le noyau phényle peut être substitué par des halogènes, des groupes alkyle en C 1-C 4, alcoxy en C 1-C 4, halogénoalcoxy en C 1-C 4, nitro ou cyano ; un groupe (alkyle en C 1-C 4)-carbonyle, un groupe (alkyle en C 1-C 4)-thiocarbonyle, un groupe alkylsulfonyle en C 1-C 4 ou (alkyle en C 1-C 4)-carbamoyle, ainsi que les stéréoisomères et énantiomères optiques de ces

dérivés.

**14.** La 2-chloro-4-fluoro-5-(N-3,4,5,6-tétrahydrophtalimido)-acétophénonoxime selon revendication 1.

**15.** La 4-fluoro-5-(N-3,4,5,6-tétrahydrophtalimido)-acétophénonoxime selon revendication 1.

**16.** L'éther méthoxy-carbonyl-éthylique-1 de la 2-chloro-4-fluoro-5-(N-3,4,5,6-tétrahydrophtalimido)-acétophénonoxime selon revendication 1.

**17.** L'éther méthoxy-carbonyl-éthylique-1 de la 4-fluoro-5-(N-3,4,5,6-tétrahydrophtalimido)-acétophénonoxime selon revendication 1.

**18.** L'éther méthylique de la 2-chloro-4-fluoro-5-(N-3,4,5,6,-tétrahydrophtalimido)-acétophénonoxime selon revendication 1.

**19.** L'éther éthylique de la 2-chloro-4-fluoro-5-(N-3,4,5,6,-tétrahydrophtalimido)-acétophénonoxime selon revendication 1.

**20.** L'éther n-butylique de la 2-chloro-4-fluoro-5-(N-3,4,5,6-tétrahydrophtalimido)-acétophénonoxime selon revendication 1.

**21.** L'éther tert-butylique de la 2-chloro-4-fluoro-5-(N-3,4,5,6-tétrahydrophtalimido)-acétophénonoxime selon revendication 1.

**22.** L'éther benzylique de la 2-chloro-4-fluoro-5-(N-3,4,5,6-tétrahydrophtalimido)-acétophénonoxime selon revendication 1.

**23.** L'éther 2-chlorobenzylique de la 2-chloro-4-fluoro-5-(N-3,4,5,6-tétrahydrophtalimido)-acétophénonoxime selon revendication 1.

**24.** L'éther 2-chlorobenzylique de la 2,4-difluoro-5-(N-3,4,5,6-tétrahydrophtalimido)-acétophénonoxime selon revendication 1.

**25.** Dérivés du N-phényl-3,4,5,6-tétrahydrophtalimide selon revendication 1, de formule Ic

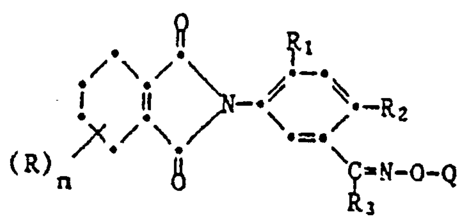

(Ic)

dans laquelle n est égal à 0, $R_3$ représente un groupe cyano, Q représente un groupe alkyle en C 1-C 10 non substitué ou substitué par des halogènes ou des groupes (alcoxy en C 1-C 6)-carbonyle ; un groupe alcényle en C 3-C 8 ou halogénoalcényle ; un groupe benzoyle ou halogénobenzoyle, et $R_1$ et $R_2$ ont les significations indiquées dans la revendication 1.

**26.** Le cyanure de méthoximino-[2-chloro-4-fluoro-5-(N-3,4,5,6-tétrahydrophtalimido)]-benzoyle selon revendication 1.

**27.** Le cyanure d'éthoximino-[2-chloro-4-fluoro-5-(N-3,4,5,6-tétrahydrophtalimido)]-benzoyle selon revendication 1.

**28.** Le cyanure d'isopropoximino-[2-chloro-4-fluoro-5-(N-3,4,5,6-tétrahydrophtalimido)]-benzoyle selon revendication 1.

29. Le cyanure de sec-butoximino-[2-chloro-4-fluoro-5-(N-3,4,5,6-tétrahydrophtalimido)]-benzoyle selon revendication 1.

30. Le cyanure de méthoxycarbonyl-éth-1-oximino-[2-chloro-4-fluoro-5-(N-3,4,5,6-tétrahydrophtalimido)]-benzoyle selon revendication 1.

31. Le cyanure d'isopropoxycarbonyl-éth-1-oximino-[2-chloro-4-fluoro-5-(N-3,4,5,6-tétrahydrophtalimido)]-benzoyle selon revendication 1.

32. Procédé de préparation des dérivés du 5-(N-3,4,5,6-tétrahydrophtalimido)-phénones de formule I, revendication 1, caractérisé en ce que l'on fait réagir l'anhydride d'un acide 3,4,5,6-tétrahydrophtalique de formule II

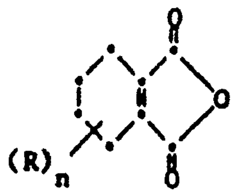

(II)

dans laquelle R et n ont les significations indiquées dans la revendication 1, dans un solvant ou diluant organique inerte, éventuellement en présence d'une petite quantité d'un agent déshydratant, avec la quantité équimoléculaire d'un dérivé de 3-aminophénone de formule V

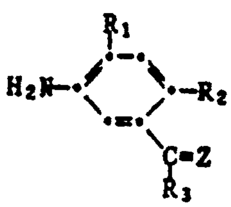

(V)

dans laquelle $R_1$, $R_2$, $R_3$ et Z ont les significations indiquées dans la revendication 1.

33. Procédé de préparation des dérivés du 3,4, 5,6-tétrahydrophtalimide de formule Ia, revendication 2, caractérisé en ce que l'on fait réagir l'anhydride d'un acide 3,4,5,6-tétrahydrophtalique de formule II

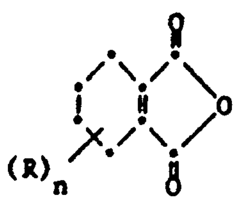

(II)

dans laquelle R et n ont les significations indiquées en référence à la formule I, dans un solvant ou diluant organique inerte, éventuellement en présence d'une petite quantité d'un agent déshydratant, avec la quantité équimoléculaire d'un dérivé 3-aminobenzoylique de formule III

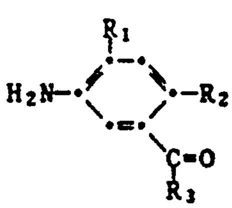

(III)

dans laquelle $R_1$, $R_2$ et $R_3$ ont les significations indiquées dans la revendication 1, et le cas échéant, lorsque Z a une signification autre que l'oxygène, on fait réagir ce produit dans un solvant inerte, en présence d'un acide, avec un alcool ou diol de formule respective IV et IVa

$R_5$-OH (IV) et HO-$R_5$-$R_6$-OH (IVa)

dans lesquelles $R_5$ et $R_6$ ont les significations indiquées dans la revendication 1.

**34.** Procédé de préparation des dérivés du N-phényl-3,4,5,6-tétrahydrophtalimide de formule Ic, revendication 15, caractérisé en ce que l'on fait réagir un sel de formule VII

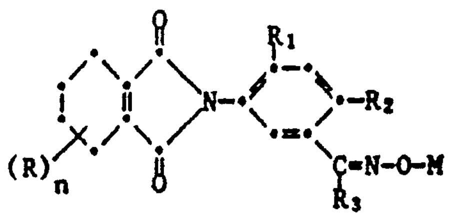

(VII),

dans laquelle M représente un cation de métal alcalin ou alcalino-terreux et n, R, $R_1$, $R_2$ et $R_3$ ont les significations indiquées ci-dessus, avec un ester réactif de formule VIII

Y - Q (VIII)

dans laquelle Q a les significations indiquées en référence à la formule I et Y représente un radical d'acide organique ou minéral.

**35.** Un produit herbicide et régulateur de la croissance des végétaux, caractérisé en ce qu'il contient en tant que substance active, avec des véhicules et/ou d'autres additifs, un dérivé d'oxime du N-phényl-3,4,5,6-tétrahydrophtalimide de formule I, revendication 1.

**36.** L'utilisation d'une substance active selon revendication 1 ou d'un produit contenant une telle substance active pour la lutte contre les croissances de végétaux indésirables.

**37.** L'utilisation d'une substance active selon revendication 1 ou d'un produit contenant une telle substance active pour l'inhibition de la croissance des végétaux.

**38.** L'utilisation d'une substance active selon revendication 1 ou d'un produit contenant une telle substance active pour agir sur la croissance des végétaux en vue d'une augmentation de rendement.

**39.** Procédé pour combattre sélectivement en prélevée ou en post-levée les végétaux adventices dans les cultures de végétaux utiles, caractérisé en ce que l'on traite les cultures de végétaux utiles ou l'aire de culture par une quantité efficace d'une substance active de formule I, revendication 1, ou d'un produit contenant un tel composé.

**40.** Procédé pour inhiber la croissance de végétaux au-delà du stade 2 feuilles, caractérisé en ce que l'on traite les végétaux durant leur croissance par une quantité efficace d'une substance active de formule I, revendication 1, ou d'un produit contenant une telle substance active.

**41.** En tant que produits intermédiaires, les 3-aminophénonoximes de formule Va

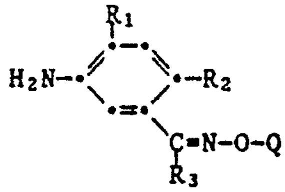

(Va)

dans laquelle

$R_1$ représente le fluor,

$R_2$ représente le chlore ou le brome,

$R_3$ représente un groupe cyano,

Q représente l'hydrogène, un ion de métal alcalin, un groupe alkyle en C 1-C 10 non substitué ou substitué par des halogènes, des groupes cyano, alcoxy en C 1-C 6 ou (alcoxy en C 1-C 6)-carbonyle ; un groupe cycloalkyle en C 3-C 8 ; un groupe alcényle en C 3-C 8 ou halogénoalcényle en C 3-C 8 ; un groupe benzoyle ou halogénobenzoyle.

**42.** Le cyanure de méthoximino-5-amino-2-chloro-4-fluorobenzoyle selon revendication 41.

**43.** Le sel de sodium du cyanure d'oximino-5-amino-2-chloro-4-fluorobenzoyle selon revendication 41.

**44.** Procédé de préparation des 3-aminophénonoximes de formule Va selon revendication 41, caractérisé en ce que l'on traite un cyanure de benzyle portant les substituants $R_1$ et $R_2$ et répondant à la formule Vb

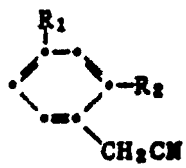

(Vb)

dans laquelle $R_1$ et $R_2$ ont les significations indiquées dans la revendication 41, dans un solvant ou diluant organique inerte, en présence d'éthylate de sodium, par le nitrite d'isopentyle, ce qui donne le sel de sodium de cyanacétoxime de formule Vc

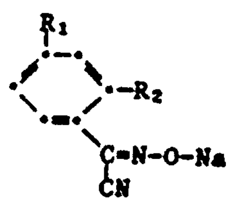

(Vc)

dans laquelle $R_1$ et $R_2$ ont les significations indiquées dans la revendication 41, qu'on éthérifie éventuellement in situ à l'aide d'un halogénure de formule

Q - Hal

dans laquelle Hal représente un atome d'halogène et Q a les significations indiquées dans la revendication 41, ce qui donne l'éther de phénonoxime de formule Vd

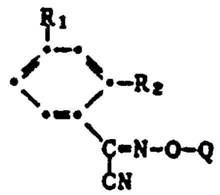

(Vd)

dans laquelle $R_1$, $R_2$ et Q ont les significations indiquées dans la revendication 41, qu'on traite par l'acide nitrique, ce qui donne l'éther de 3-nitrophénonoxime de formule Ve

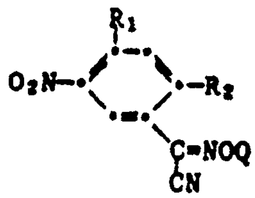

(Ve)

dans laquelle $R_1$, $R_2$ et Q ont les significations indiquées dans la revendication 41, qu'on réduit dans l'acide chlorhydrique aqueux, par le chlorure d'étain, en l'éther de 3-aminophénoxime de formule Va de la revendication 41.